Europäisches Patentamt

European Patent Office

Office européen des brevets

(19)

(11) Publication number: **0 258 899**
**A2**

(12)

# EUROPEAN PATENT APPLICATION

(21) Application number: 87112893.0

(22) Date of filing: 03.09.87

(51) Int. Cl.⁴: **C12Q 1/02** , **C12Q 1/68** ,
**G01N 33/68** ,
//C12N15/00,C12P21/00,G01N3-
3/577,G01N33/569

(30) Priority: 05.09.86 US 904965

(43) Date of publication of application:
09.03.88 Bulletin 88/10

(84) Designated Contracting States:
AT BE CH DE ES FR GB GR IT LI LU NL SE

(71) Applicant: **MEDICAL RESEARCH COUNCIL**
**20 Park Crescent**
**London W1 4AL(GB)**

(72) Inventor: **Grosveld, Frank**
**70 Salcombe Gardens**
**London, NW7 2NT(GB)**
Inventor: **Burke, Julian Francis**
**6 Friars Walk**
**Lewes East Sussex, BN7 2LE(GB)**
Inventor: **Eccles, Sarah Jane**
**38 Glenhurst Avenue**
**London, NW5 1PS(GB)**
Inventor: **Wrighton, Christopher J.**
**Hunt Lodge 85 Winnington Road**
**Hampstead Garden Suburb London N2(GB)**
Inventor: **Vidal, Miguel-Angel**
**Flat 4 14 Seymour Road**
**London N3 2NH(GB)**

(74) Representative: **Henkel, Feiler, Hänzel &**
**Partner**
**Möhlstrasse 37**
**D-8000 München 80(DE)**

(54) Mammalian cell mutagenicity screening test.

(57) A method for testing a substance to determine whether the substance is mutagenic is described. The method involves contacting with the substance a mammalian tester cell, the cell comprising DNA carrying a mutation which renders the DNA incapable of directing production of a detectable marker but which is capable of undergoing back mutation so that a detectable marker can be produced under conditions which permit back mutation of the DNA, treating the resulting tester cell under conditions which permit back mutation of the DNA and production of the detectable marker, and detecting the presence of the detectable marker, such presence indicating that the substance is mutagenic. The invention also provides a mammalian tester cell comprising foreign DNA carrying a mutation which renders the DNA incapable of directing production of a detectable marker but which is capable of undergoing back mutation so that the detectable marker can be produced.

## MAMMALIAN CELL MUTAGENICITY SCREENING TEST

Background of the Invention

Throughout this application various publications are referenced by arabic numerals within parentheses. Full citations for these references may be found at the end of the specification immediately preceding the claims. The disclosures of these publications in their entireties are hereby incorporated by reference into this application in order to more fully describe the state of the art as known to those skilled therein as of the date of the invention described and claimed herein.

In vivo tests to demonstrate whether a substance has mutagenic properties are expensive and time-consuming. They require large numbers of laboratory animals, animal care facilities, maintenance, personnel, and other costly items. It is desirable to have fast and inexpensive in vitro tests of mutagenicity to identify compounds for subsequent testing to determine its mutagenicity in vivo.

Several tests using mammalian cells have been described. Many of these tests are based on rendering a functional gene non-functional and rely on the selection of mutant colonies which are drug resistant due to inactivation of various enzymes. Many of these tests involve forward mutation systems. Forward mutations have the disadvantage that they may not detect rare mutational events which occur against a background of frequent mutational events. Such tests are lengthy, taking two to three weeks, and expensive (1).

Another type of known test involves chromosomes. It involves comparison between the frequency of sister chromatid exchange in control cells and in cells treated with the substance being tested. The basis for chromatid exchanges remains uncertain, except that there must be breaks and rejoinings to produce the altered pattern seen in stained preparations. Many mutagens induce chromosomal breakage leading to structural rearrangements, but whether sister chromatid exchanges arise by the same processes is not known at the present time. One of the difficulties with the sister chromatid exchange test is the uncertainty regarding the amount, if any, of genetic damage arising in cells with high amounts of sister chromatid exchanges. Test results do not indicate whether mutations and other genetic effects are induced or whether chromatid exchanges simply occur unaccompanied by genetic or inherited alteration. Evidence suggests, however, that an increase in the frequency of sister chromatid exchanges is accompanied by an increase in genetic damage and in mutation rate (2).

The Ames test (3) has been shown to be very sensitive and to give a good quantitative correlation between mutagenicity and carcinogenicity for a wide range of chemicals (4).

The bacterium Salmonella typhimurium is the test organism used in the Ames test. The bacterial strain carries a cell wall mutation that permits most chemicals to enter the cells readily, a uvr mutation that abolishes most excision repair, a plasmid carrying some unknown factor that exerts mutator activity in Salmonella so that DNA damage is converted into mutations with high frequency, and a genetic requirement for the amino acid histidine. The his⁻ mutation can be reverted back to his⁺ by either base substitution or frameshift mutations. A mixture of cytoplasmic ingredients obtained as a cell-free extract from a rat liver is also added to the culture dishes. The enzymes in this rat liver fraction can convert test chemicals to other products, some of which may be mutagenic even if the original substance is not. Many carcinogenic chemicals are not harmful until they have been metabolized to other products in the mammalian system, and the rat liver extract can accomplish these changes in the culture medium. The assay involves scoring for his⁺ revertants (or suppressor mutants which cause a pseudowild phenotype) which appear as colonies on minimal media.

If a substance is found to be mutagenic it is likely also a carcinogen. Once the substance is tested in animals such as mice, it can definitely be determined whether tumors will be produced. The Ames test reverse mutation system measures the reversion or suppression of specific preexisting mutations and therefore gives more precise information about the nature of the mutational changes caused by a particular compound.

There are shortcomings to using a bacterial test such as the Ames test to identify substances having mutagenic properties. As with most bacterial tests, plate counting techniques are used to determine the number of revertant bacteria. For the Ames test, plate counting requires a 48 hour growth period, plus time to count both the test plates and control plates used to monitor spontaneous reversion. Additionally, certain mutagenic chemicals will not be identified as having mutagenic properties because of the fundamental differences in organization and metabolism between bacterial and mammalian cells. The Ames test measures single gene mutations in bacteria. Mammalian cells, however, are prone to chromosomal defects such as aneuploidy breaks and translocations. Such lesions could be missed in any screening test that utilizes marker genes carried on chromosomes that also contain large numbers of other genes needed for reproduction. Multigene deletions or complete loss of such chromosomes would cause failure of the affected cell to form a colony and would, therefore, not be recorded.

Findl et al., United States Patent No. 4,256,832 (1981) disclose a method of screening potential carcinogens or mutagens which includes contacting a mutant strain of eurcaryotic or prokaryotic tester organism capable of reverting to a normal form in the presence of a carcinogen or mutagen with a substance to be screened for mutagenic properties, growing the tester organism, and detecting the consumption of oxygen to determine if the tester organism has reverted to its normal form, and thereby determine if the material to be screened is potentially carcinogenic or mutagenic.

There are shortcomings to using the Findl et al. method to identify substances having mutagenic properties. The method requires an apparatus including an oxygen electrode for generating an electrical signal proportional to the amount of oxygen detected. When one of the criteria for testing the effects of unknown sub stances involves the measurement of oxygen consumption, there is always the possibility that the unknown substance is an uncoupler of oxidative phosphorylation. If this is the case a very rapid increase in oxygen uptake will be registered. This oxygen uptake, however, is independent of "real" respiration and no adenosine triphosphate will be produced. Thus, any substance that produces an increase in oxygen uptake, independent of increased numbers of organisms, must first be tested for uncoupling activity.

The present invention overcomes these shortcomings by providing a method for testing a substance to determine whether the substance is mutagenic using a mammalian tester cell. Thus, this invention will permit identification of mutagenic substances which the Ames test will fail to identify. Additionally, the present invention does not require the measurement of oxygen consumption to identify mutagenic properties or an apparatus for detecting oxygen consumption, and substances being tested for mutagenicity need not be tested first for uncoupling activity. The present invention also eliminates the need for a colony growth step thereby permitting much faster measurements than prior methods. Thus, this invention permits detection not only of mutagens which are not toxic, but also mutagens which are toxic, to cell growth. This invention also allows direct confirmation of the existence of a particular mutation by isolating, e.g. by replica plating or FACS®. (Beckton Dickinson and Company, Paramus, NJ), clonal populations of cells carrying the induced mutation. Finally, in a preferred embodiment, this invention provides a genetically engineered tester cell which comprises foreign DNA not normally present in a mammalian cell. The invention thus permits an optimum tester cell to be constructed and does not require reliance upon naturally occurring cells or mutants readily obtainable therefrom.

## Summary of the Invention

The invention is a method for testing a substance to determine whether the substance is mutagenic. The method comprises contacting with the substance a mammacelian tester cell comprising DNA carrying a mutation which renders the DNA incapable of directing production of a detectable marker but which is capable of undergoing back mutation so that a detectable marker can be produced, under conditions which permit back mutations of the DNA; treating the resulting tester cell under conditions which permit production of the detectable marker, and; detecting the presence of the detectable marker, such presence indicating that the substance is mutagenic. The treatment step is carried out during a period of time such that cell doubling of the test cell occurs no more than about three times.

The invention also is a mammalian tester cell comprising foreign DNA carrying a mutation which renders the DNA incapable of directing production of a detectable marker but which is capable of undergoing back mutation so that the detectable marker can be produced.

The invention also is a human tester cell comprising foreign DNA carrying a mutation which renders the DNA incapable of directing production of a detectable marker but which is capable of undergoing back mutation so that the detectable marker can be produced.

The invention also is a hypermutagenic mammalian tester cell comprising foreign DNA carrying a mutation which renders the DNA incapable of directing production of a detectable marker but which is capable of undergoing back mutation so that the detectable marker can be produced.

The invention also is a mammalian tester cell comprising multiple copies of foreign DNA, each copy carrying a mutation which renders the DNA incapable of directing production of a detectable marker but which is capable of undergoing back mutation so that the detectable marker can be produced.

The invention also is a mammalian tester cell comprising foreign DNA carrying a mutation which renders the DNA incapable of directing production of a cell surface protein but which is capable of undergoing back mutation so that the cell surface protein can be produced.

## Brief Description of the Figures

Fig. 1 shows the sequence of amber and frameshift mutagenic oligonucleotides showing nucleotide changes introduced by site specific mutagenesis.

Fig. 2 shows a restriction map of the 5KB HindII fragment, containing the H-2K$^b$ gene. The 340bp fragment which was subcloned into M13 mp9 for mutagenesis is shown above the map and the position of insertion of RSV LTR sequence is indicated.

Fig. 3 shows the 340bp HindII/SmaI fragment from the 5' region of the H-2K$^b$ gene subcloned into M13 mp9 to provide a template for mutagenesis.

Fig. 4 shows the detection of H-2K$^b$ RNA in transient assays.

Fig. 5 shows a restriction map of the cosmid p228, including the modification introduced at the SalI site to produce pSCM 100.

Fig. 6 shows the staining of H-2K$^b$ expressing cells in the in situ assay.

Fig. 7 shows FACS profiles of mixtures of L cells positive and negative for expression of H-2K$^b$.

Fig. 8 shows a Southern blot of DNA from various L cell clones containing H-2K$^b$ amber (am) or frameshift ($\Delta$) genes probed with a $^{32}$P labelled 500bp fragment from the RSV LTR. Plasmid DNA representing 1 and 10 copies per genome are shown as standards.

Fig. 9 shows putative revertant cells detected by in situ staining after UV mutagenesis and sorting of Lam13 cells.

A. shows cells grown for 3 days after sorting before in situ analysis

B. shows cells grown for 5 days after sorting before in situ analysis.

Fig. 10 shows the plasmid p292 differs from p291 in that the Bam HI - Sal I fragment has been deleted and Cla I linker has been inserted keeping both Bam HI and Sal I sites.

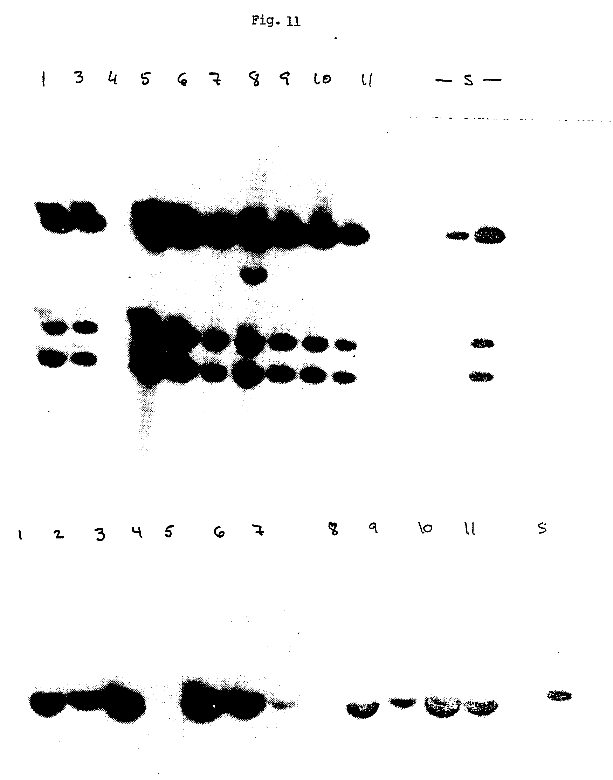

Fig. 11 shows a Southern blot analysis of HeLa clones transfected with p292-K$^b$ plasmids.

A. shows low molecular weight DNA from different clones (2 $\times$ 10$^6$ cells/sample) digested with Bam HI and probed with the whole p292-K$^b$ plasmid. On the right (S) different amounts of p292-K$^b$ plasmid equivalent to 1, 3 and 10 copies/cell.

B. shows genomic DNA (9 micrograms) digested with Bgl II (an enzyme which linearizes p292-K$^b$ plasmids). The probe is p228 (page 30). On the right (S) 10 copies/cell equivalent of p292-K$^b$ plasmid.

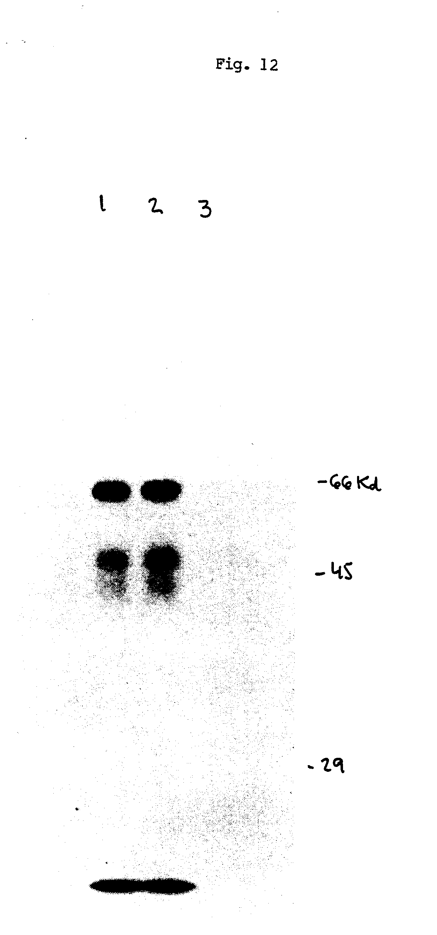

Fig. 12 shows the immunoprecipitation of H-2K$^b$ molecule by the monoclonal antibody MV3. Precleared lysates of cell surface radioiodinated H-2K$^b$ transfected L cell IC4 clones were incubated with MV3 hybridoma supernatants or an irrelevant antibody. Immunocomplexes were then isolated by incubation with rabbit IgG anti-mouse IgG - coated protein A-sepharose and analysed by SDS-polyacrylamide gel electroporesis.

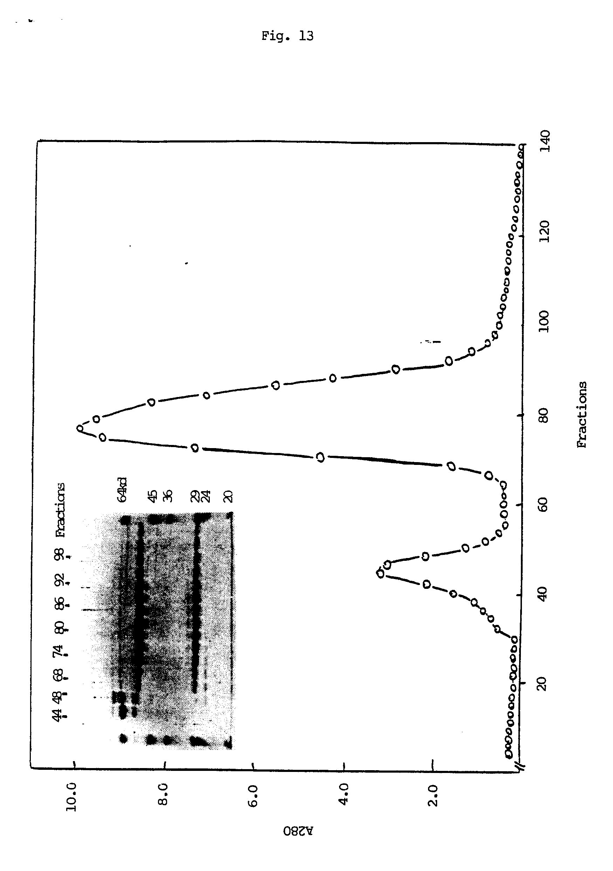

Fig. 13 shows the purification of the anti-H-2K$^b$ monoclonal antibody MV3. 15ml of ascitic fluid were loaded on a 2$\times$18cm column of BioGel HPT equilibrated with 0.01M sodium phosphate buffer, pH 6.8. Fractions correspond to the protein eluted by a linear gradient from 0.01-0.3M sodium phosphate buffer pH 6.8, at 100ml hr$^{-1}$, size of fractions is 3.2ml. An electrophoretic analysis of reduced samples of indicated fractions in an SDS-polyacylamide gel is also shown. On the right side of the reduced samples the size of molecular weight markers is shown.

## Detailed Description of the Invention

The invention comprises a method for testing a substance such as a chemical to determine whether the substance is mutagenic, i.e., whether it acts in vivo on DNA to mutate or otherwise change the DNA, particularly so as to impair or otherwise effect the functioning of the DNA. The method comprises contacting, such as by an aqueous or other adding solution containing the substance, e.g. in dissolved or suspended form to cells in tissue culture or by placing cells in tissue culture in an environment containing the substance in vapor phase, a mammalian tester cell, e.g. cells derived from human or animal subjects, with the substance to be tested. The tester cell comprises DNA carrying a mutation which renders the DNA incapable of directing production within the cells of a detectable marker, e.g. a direct or indirect gene product of the DNA such as a protein or other polypeptide or a metabolite where production is catalyzed by an enzyme encoded by the DNA. This DNA is capable of undergoing back mutation so that the detectable marker is produced, and the contacting is effected under conditions which permit back mutations of the DNA. Concurrently with or subsequent to contacting the tester cell with the substance being tested, the resulting tester cell is treated during a period of time such that cell doubling of the tester cell occurs no more than about three times under conditions which permit production of the detectable marker if back mutation of the DNA has occurred. Appropriate conditions for treating the tester cell will vary depending upon the nature and identity of the detectable marker. Thus, if the detectable marker may be detected only after limited cell doubling treatment must include growth conditions permitting such limited growth. By contrast, if the detectable marker may be detected directly in the tester cell perse treatment will comprise growth, but may require contacting the tester cell with a material in the presence of which the detectable marker may be observed. Finally, the presence of the detectable marker may be accomplished using conventional techniques such as automated or manual microscopy, fluorescence, luminescence, colorimetry and cell sorting, such presence indicating that the substance being tested is mutagenic.

The DNA carrying the mutation may desirably be present in the tester cell in multiple copies, for example greater than 10 or more copies. The DNA is preferably foreign DNA, i.e. DNA not normally present in the test cell and may be procaryotic, eucaryotic, chromosomal, extra-chromosomal, or synthetic.

The mammalian tester cell is desirably of human origin so as to optimize the probability that substances tested and determined to be mutagenic or nonmutagenic will exhibit the same property in vivo in humans. However, one skilled in the art would recognize that the tester cell may be any animal cell or an insect cell. In one embodiment the human cells are Hela cells. Also, desirably the tester cell is a hepatoma (liver) cell because of the correlation between human carcinogenesis and liver dysfunction. Suitable tester cells may be adherent or nonadherent but in either case are preferably hypermutagenic. Typical properties of cells useful as tester cells include the following: deficiency in at least one DNA repair enzyme, e.g. an in excission repair enzyme; hypersensitivity to ultraviolet radiation, ionizing radiation, alkylating agents, cross-linking compounds, or chemical carcino gens. In addition to human tester cells, such as human hepatoma cells, other suitable tester cells include Xeroderma pigmentosum and Ataxia telangiectasia and L cells. The mammalian tester cell desirably has a high frequency of chromosome aberrations or a high frequency of spontaneous sister chromatid exchanges. Finally, the mammalian tester cell line may have an altered nucleotide pool size.

In certain embodiments of the invention the contacting step is carried out in the presence of metabolic activators, e.g. liver enzymes which may be present in the form of a liver extract.

The DNA may encode a selectable marker for growth of the tester cell, a selectable marker for growth of bacterial cells which comprise the DNA, or a selectable marker for growth of both the tester cell and bacterial cells which comprise the DNA.

The mutation may be any of the numerous types of mutations known to occur. For example, the mutation may be in a stop codon, may involve a base substitution, insertion or deletion, may be a gene duplication or may be a point or frame-shift mutation. The mutation may be in the coding sequence of the DNA, in a regulatory region associated with the DNA, e.g. a promoter sequence, or in an RNA processing sequence associated with the DNA.

The detectable marker may be RNA, e.g. mRNA, a protein or other polypeptide or a metabolate or other substance produced by the tester cell. Examples of proteins include enzymes and cell surface proteins. The detectable marker may be detected using conventional tech niques including microscopy, fluorescence, luminescence, colorimetry, cell sorting or an antibody or other reagent directed to the marker. The detecting step may be manual or automated and may comprise replica plating or visualizing single cells. In a perfered embodiment, the automated detection is determined by the use of a florescence activated cell sorter.

The invention also comprises a method for testing a substance such as a chemical to determine whether the substance is mutagenic, i.e., whether it acts in vivo on DNA to mutate or otherwise change the DNA, particularly so as to impair or otherwise effect the functioning of the DNA. The method comprises contacting, such as by an aqueous or other adding solution containing the substance, e.g. in dissolved or suspended form to cells in tissue culture or by placing cells in tissue culture in an environment containing the substance in vapor phase, a mammalian tester cell, e.g. cells derived from human or animal subjects, with the substance to be tested. In a preferred embodiment of the invention, the tester cell comprises foreign DNA carrying a mutation which renders the DNA incapable of directing production within the cells of a detectable marker, e.g. a direct or indirect gene product of the DNA such as a protein or other polypeptide or a metabolite where production is catalyzed by an enzyme encoded by the DNA. This DNA is capable of undergoing back mutation so that the detectable marker is produced, and the contacting is effected under conditions which permit back mutations of the DNA. Tester cells having foreign DNA provide

5

greater flexibility in DNA sequence and design changes. Concurrent with or subsequent to contacting the tester cell with the substance being tested, the resulting tester cell is treated under conditions which permit production of the detectable marker if back mutation of the DNA has occurred. Appropriate conditions for treating the tester cell will vary depending upon the nature and identity of the detectable marker. Thus, if the detectable marker may be detected only after limited cell doubling, treatment must include growth conditions permitting such limited growth. By contrast, if the detectable marker may be detected directly in the tester cell per se treatment will comprise growth, but may require contacting the tester cell with a material in the presence of which the detectable marker may be observed. Finally, the presence of the detectable marker may be accomplished using conventional techniques such as automated or manual microscopy, fluorescence, luminescence, colorimetry and cell sorting, such presence indicating that the substance being tested is mutagenic.

The invention also comprises a method for testing a substance such as a chemical to determine whether the substance is mutagenic, i.e., whether it acts in vivo on DNA to mutate or otherwise change the DNA, particularly so as to impair or otherwise effect the functioning of the DNA. The method comprises contacting, such as by an aqueous or other adding solution containing the substance, e.g. in dissolved or suspended form to cells in tissue culture or by placing cells in tissue culture in an environment containing the substance in vapor phase, a mammalian tester cell, e.g. cells derived from human or animal subjects, with the substance to be tested. In a preferred embodiment of the invention the tester cell comprises foreign DNA carrying a mutation which renders the DNA incapable of directing production within the cells of a detectable marker, e.g. a direct or indirect gene product of the DNA such as a protein or other polypeptide or a metabolite where production is catalyzed by an enzyme encoded by the DNA. Tester cells having foreign DNA provide greater flexibility in DNA sequence and design changes. This DNA is capable of undergoing back mutation so that the detectable marker is produced, and the contacting is effected under conditions which permit back mutations of the DNA. Concurrently with or subsequent to contacting the tester cell with the substance being tested, the resulting tester cell is treated under conditions which permit production of the detectable marker if back mutation of the DNA has occurred. Appropriate conditions for treating the tester cell will vary depending upon the nature and identity of the detectable marker. Thus, if the detectable marker may be detected only after limited cell doubling treatment must include growth conditions permitting such limited growth. By contrast, if the detectable marker may be detected directly in the tester cell per se, treatment will comprise growth, but may require contacting the tester cell with a material in the presence of which the detectable marker may be observed. The foreign DNA also encodes a selectable marker for growth of the tester cell. Tester cells having foreign DNA which encodes a selectable marker for growth of the tester cell allow for greater ease in purification and development of the marker. Finally, the presence of the detectable marker may be accomplished by using conventional techniques such as automated or manual microscopy, fluorescence, luminescence, colorimetry and cell sorting, such presence indicating that the substance being tested is mutagenic.

The invention also is a human tester cell comprising foreign DNA carrying a mutation which renders the DNA incapable of directing production within the cells of a detectable marker e.g. a direct or indirect gene product of the DNA such as a protein or other polypeptide or a metabolite where production is catalyzed by an enzyme encoded by the DNA. Tester cells having foreign DNA provide greater flexibility in DNA sequence and design changes. This DNA is capable of undergoing back mutation so that the detectable marker is produced. The human tester cell may be hypermutagenic. The human tester cell may be comprised of multiple copies of DNA for example, greater than 10 or more copies, each copy carrying a mutation. The detectable marker may be a cell surface protein.

The invention also includes a mammalian tester cell comprising foreign DNA carrying a mutation which renders the DNA incapable of directing production within the cells of a detectable marker. Tester cells having foreign DNA provide greater flexibility in DNA sequence and design changes. This DNA is capable of undergoing back mutation so that the detectable marker is produced. The foreign DNA may be mammalian DNA, bacterial DNA, viral DNA, plant DNA or fungal DNA. Alternatively, the invention provides a mammalian tester cell comprising foreign RNA carrying a mutation which renders the DNA incapable of directing production within the cells of a detectable marker. The DNA or RNA may additionally encode a selectable marker for growth of the tester cell, a selectable marker for growth of bacterial cells which comprise the DNA, or for a selectable marker growth of both the tester cell and bacterial cells which comprise the DNA. The mammalian tester cell may be hypermutagenic. The cell may be comprised of multiple copies of DNA, for example, greater than 10 or copies each copy carrying a muta tion. The detectable marker may be a cell surface protein.

Although the text of the subject application concerns in vitro and in situ mammalian tester cells, those skilled in the art will recognize that the methods of this invention may be used in vivo, e.g. the genes used in the tissue culture system can be put into transgenic mice and expressed, for example, the liver. This allows rodents to be treated with chemicals for long periods of time and the genotoxic effects measured in vivo. It is likely that reversion of a mutation in such a transgenic animal could be detected at an early stage after mutagen treatment.

According to one embodiment of the present invention, specific mutations are introduced into the N-terminal leader sequence encoding the signal peptide of the mouse H-2K$^b$ gene (5) using oligonucleotide directed site specific mutagenesis. Single or multiple copies of these modified genes are then introduced into one or more mammalian cell lines where they serve as targets for the action of potential mutagens. After a short expression period (up to 4-5 days), the mutagen treated cells are assayed for expression of H-2K$^b$ on the cell surface using a monoclonal antibody. A cell which contains a gene copy in which the mutation has been corrected produces a protein product which is detectable on the cell surface. The number of H-2K$^b$ positive cells provides a direct measure of the mutagenic activity of the test compound. The inclusion of multiple copies of the tester gene in each cell increases the target size. The increased target size reduces the number of cells which have to be screened.

The H-2K$^b$ gene is a suitable choice as a target molecule for mutagenesis because its entire coding sequence is known (5), it encodes a cell surface molecule which is therefore accessible to antibodies, and antibodies which recognize this molecule have been described (6).

Four mutants of the H-2K$^b$ gene are included in the description of the present invention. The first contains an amber mutation, the second a missense mutation. Between them, these constructs can pick up every possible base substitution. The third contains a two base pair mutation (and requires a frameshift for reversion). The fourth mutation contains a partial gene duplication (and requires a deletion or recombination for reversion). The first three mutants lie in the first exon of the gene which encodes the signal peptide. The amber and frameshift genes have been introduced into mammalian cell lines and have been shown to be transcribed. They have been shown not to direct the synthesis of any detectable H-2K$^b$ protein.

In order to introduce multiple copies of the mutant genes into tester cell lines, an autonomously replicating Epstein Barr virus based vector has been used. Cells expressing the EBNA-1 nuclear antigen support the autonomous replication of vectors bearing the oriP sequence of the Epstein Barr virus genome. Human cell lines carrying such vectors have been reported to contain up to 100 plasmid copies per cell (7). Introduction of multiple copies of the mutant H-2K$^b$ genes into such a vector, followed by transfection into cell lines expressing EBNA-1, yields cells bearing a large number of copies of the target genes. A further advantage of using a vector which can replicate independently is that it simplifies the recovery of revertant genes generated in the mutation test.

Immunochemical techniques are currently among the most powerful approaches available for the detection of rare molecules in complex samples such as cell populations. For instance, immunochemical procedures have been used to screen cDNA libraries (8), to detect antigen-positive cells in cell populations transfected with total genomic DNA (9) and to isolate rare cells bearing variants of a particular antigen (10). In order to adopt such an approach for the mutagenicity test described herein it has been necessary to produce a novel antibody recognizing the wild-type H-2K$^b$ molecule and to devise a sufficiently sensitive detection method. The production of mouse monoclonal antibodies against the H-2K$^b$ molecule is described hereinafter, together with the development of an in situ immunoenzymatic protocol capable of detecting a few positive cells in a large negative cell population.

The invention will be better understood from the Experimental Details which follow. However, persons skilled in the art will readily appreciate that numerous variations are within the scope of the invention.


## Experimental Details


## MATERIALS AND METHODS


### Enzymes and chemicals

All restriction enzymes were obtained from Anglian Biotechnology Ltd., U.K. Other enzymes were obtained from Bethesda Research Laboratories, Inc. The buffers and conditions recommended by the manufacturers were employed for all enzyme reactions. ClaI linkers were obtained from New England Biolabs, U.S.A. Unless stated otherwise, all chemicals and reagents were obtained from Sigma Chemical Co.

Preparation of oligonucleotides

Oligonucleotides were prepared by automated phosphoramidite chemistry using the Beckman System 1 DNA synthesizer. Reaction products were deblocked and cleaved from the resin support and 20-meric molecules isolated from the oligonucleotide mix by high pressure liquid chromatography. Oligonucleotide fractions were desalted by gel filtration through 0.2M triethyl ammonium bicarbonate (freshly diluted) and lyophilised in aliquots. Stock and working solutions of 260ng/microliter (45 micromoles oligonucleotide) and 2.6ng/microliter (0.45nM oligonucleotide), respectively, were prepared. The average $M_r$ of oligonucleotides is 6065.

Site specific mutagenesis

The gapped duplex was prepared essentially as described by Kramer et al . (11), except that the annealing mixture was allowed to cool slowly from 65°C to room temperature. Single stranded circular phage DNA prepared from M13mp9 (amber) containing the 5' H-2K$^b$ gene HindII/Smal fragment, was annealed to linear M13mp9 (wild-type) from which the polylinker had been excised and removed by EcoRI and HindIII digestion and centrifugation through a 10-30% sucrose gradient (Beckman® SW40 rotor, 30K for 16 hours at 15°C). Gapped duplex formation was confirmed by agarose gel electrophoresis and by incorporation of alpha-$^{32}$P-dATP into TCA precipitable material under the conditions used for the repair synthesis. Oligonucleotide phosphorylation, an nealing of the oligonucleotide to the gapped duplex, repair/ligation reactions and transfection of E. coli mutL were carried out according to Kramer et al. (12). MutL transfectants were picked in agar plugs and phage eluted into 200 microliters broth by vortexing and incubation at 37°C for 15 minutes. 3 microliters of this phage suspension was replica spotted onto two agar plates, one seeded with E. coli JM101 (supE) and the other seeded with E. coli W71-18 (supO). Phage infecting both strains were amplified. Replicative form DNA was prepared for restriction enzyme analysis, and single stranded DNA was prepared for dideoxy nucleotide sequencing.

Oligonucleotide directed all-round synthesis was carried out essentially as described by Zoller and Smith (13). A 20-fold molar excess of 5' phosphorylated oligonucleotide was annealed to single stranded template DNA by slow cooling from 65°C to 25°C in a beaker containing 100ml of water. The synthesis was carried out in a total volume of 100 microliters containing 6.6mm Tris-HCl (pH 7.5), 6.6mm mgCl₂, 10 mm DTT, dATP, dCTP, dGTP, dTTP and ATP (uniformly 50 micromoles), 25 units of DNA polymeraseI Klenow fragment and 30 units of T4 DNA ligase, which was incubated overnight at 16°C. After phenol and chloroform extraction, covalently closed circular DNA was purified from a 0.8% low melting point agarose gel and used to transfect E. coli MutL. Transfectants were amplified in 1 ml cultures and after removal of the bacteria centrifugation (5 minutes, Eppendorf microfuge) 5 microliters of phage suspension was applied to nitrocellulose filters using a Bio-Rad hybridot apparatus (Bio-Rad, Richmond,CA). The filter was baked at 80°C for 2 hours, then prehybridrized overnight at 67°C in 6$^×$SSC, 10$^×$ Denhardts solution (0.2% Ficoll, 0.2% BSA,0.2% polyvinylpyrrolidone) 0.2% SDS. Hybridization to the oligonucleotide probe, labelled with $^{32}$P using T4 polynucleotide kinase, was carried out at 23°C for 1 hour in prehybridization buffer containing denatured, sonicated salmon sperm DNA (100 micrograms/ml). The filter was autoradiographed after washing in 6$^×$SSC first at room temperature, then at 37°C, 46°C and 55°C (5 minutes in 50 ml of 6$^×$SSX).

Transfections

The procedure of Wigler et al. (14) was used for the preparation of calcium phosphate/DNA coprecipitates. Electroporation was carried out as described by Neumann et al. (15) and DEAE dextran facilitation of DNA uptake, as described by Sompayrac and Danna (16). In all cases cells were harvested for transient assays and selective media was introduced for stable transformants, 48 hours after transfection.

SI protection analysis

This was carried out essentially as described by Berk and Sharp (17). In each case, 15 micrograms of total cellular RNA was hybridized to a probe which had been end labelled with $^{32}$P using T4 polynucleotide kinase (18).

## Monoclonal antibodies

Hybridoma cells were produced according to standard techniques (19). P3$\times$63 Ag 8.6.5.3 and Sp2/0 Ag14 (two non-secretor Balb/c myelomas) were fused with splenocytes from B10.A(4R) mouse (which had been immunized with B10 mouse splenocytes) or from a Balb/c mouse (which had been immunized with Balb/b mouse splenocytes) using polyethylene glycol. Hybrids were selected in HAT medium and cultures with apparent colonies (after 14-21 days) were assayed for anti-H-2 antibody production. This was done using an indirect cellular radioimmunoassay in which EL4 cells (thymic leukaemia cells of b haplotype) or transfectant L cells expressing the H-2K$^b$ antigen were first incubated with hybridoma culture supernatants and thereafter with a radiolabelled anti-mouse immunoglobulin antibody. Positive cultures were then cloned by limiting dilution using a macrophage feeder layer. Positive cultures derived from wells seeded with one or 0.5 cells were chosen for further expansion.

## In situ detection assay

Clones of L-cells transfected with the cloned H-2K$^b$ gene were used as positive cells in reconstruction experiments with normal L-cells (as negative counterparts) to set up conditions for an in situ assay to detect H-2K$^b$-positive cells. A mouse monoclonal anti-H-2K$^b$ antibody already available (from ATCC) was used in this series of experiments.

Cells growing in tissue culture dishes (Dulbecco's modified MEM or alpha-MEM media supplemented with 10% newborn calf serum, antibiotics and 1 mM pyruvate) were washed twice with Dulbecco's PBS prior to fixation with 0.125% glutaraldehyde in PBS for 5 minutes at room temperature. Following two washes with PBS, cells were incubated with supernatant culture of the anti-H-2K$^b$ hybridoma for 1-2 hours at room temperature. Plates were then washed thoroughly with PBS (four times, 2-3 minutes each) and incubated with an anti-mouse immunoglobulin antibody coupled to horseradish peroxidase. This antibody was used at an appropriate dilution (1:200-1:400) in PBS containing 10% fetal calf serum and 1% bovine serum albumin. Plates were washed with PBS as above and one last wash was done with 0.05M sodi m acetate buffer pH 5.5 prior to the enzymatic reaction to develop immunocomplexes. 3-aminoethylcarbazole (AEC) (1-2mM from a stock solution in dimethylsulfoxide) was used as a substrate for horseradish peroxidase in a 0.05M sodium acetate buffer pH 5.5 containing 0.03% H$_2$O$_2$. A red-brown colored product appears at 5-10 minutes, but incubations were usually continued for a total of 30 minutes. Plates were then rinsed with PBS to wash away and remaining substrate.

## Mutagenesis and analysis on the EPICS V/FACS II cell sorter

Mutagenesis was carried out as follows: On Day 1 $5\times10^7$ -$2\times10^8$ cells were plated on 15cm dishes ($7.5\times10^6$ cells/dish) in medium supplemented with HAT. On Day 2 the medium was aspirated and the cells exposed to 254nm UV light at a total dose of 30-45 Joules,/m$^2$. The medium was replaced and the cells incubated overnight. On Day 3 cells were trypsinized and replated on 15cm dishes and aliquots were taken for estimation of cell survival by comparison with a culture treated in the same manner, but not exposed to UV light (2-5% survival at these doses). Cultures were then grown for a further 4-5 days to expand the revertant population prior to analysis. Cells were then harvested with trypsin and $2\times10^6$ cells were plated on a 10cm dish and grown for a further 2-3 days for analysis in situ. The remainder of the cells ($2-5\times10^7$) were labelled for sorting on an EPICS V cell sorter. [Cells were incubated on ice for 30 minutes with the monoclonal antibody MV3, specific for H-2K$^b$, washed twice in PBS containing 2% newborn calf serum and incubated for a further 30 minutes on ice with an FITC conjugated goat anti-mouse IgG antibody (Sigma). The second antibody was removed by dilution with PBS (+ 2% NBCS) and centrifugation, and the cells resuspended in the same buffer to which propidium iodide was added (10 micrograms/ml final concentration)]. The cells were then analysed using an EPICS V cell sorter. Cells stained with propidium iodide, which exhibit red fluorescence (PMT 540v), were gated out (dead cells) and the top 3% of cells exhibiting the highest levels of green fluorescence (PMT 720v) were collected into a 3cm dish containing HAT medium and grown for 3-4 days before fixing with gluteraldehyde and analysis by the in situ immunoenzymatic assay.

For analysis on the FACS II cell sorter, cells were labeled in the same way as described above.

Results

## Construction of amber and frameshift mutants of the H-2K^b gene

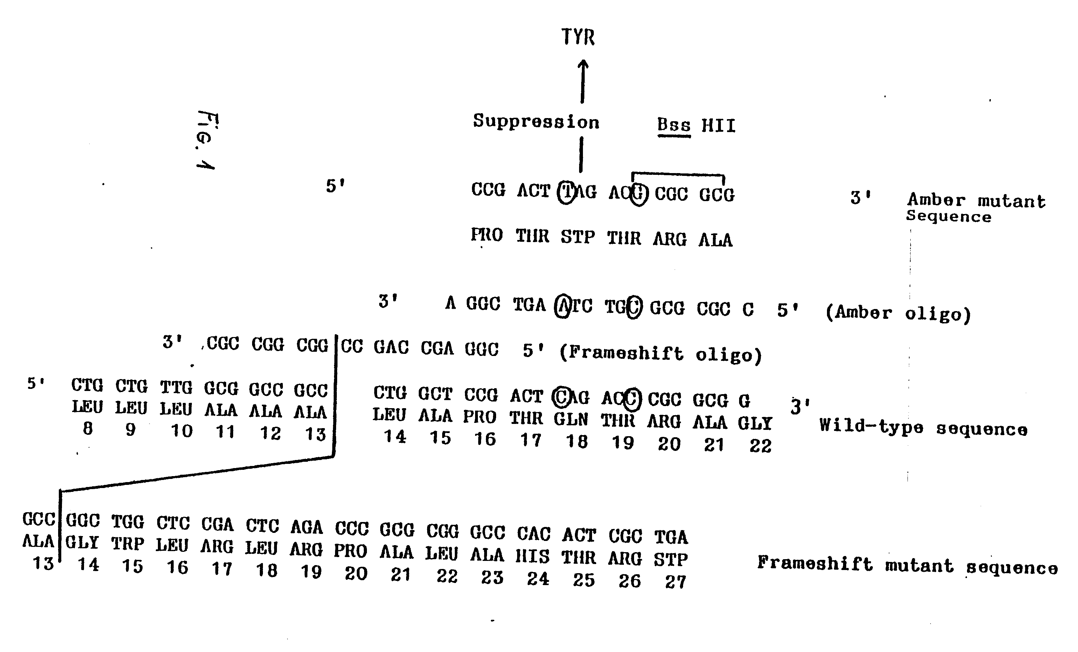

Using the published DNA sequence of the H-2K$^b$ gene (4), two oligonucleotide primers were designed which would introduce specific mutations into exon 1 (encoding the signal peptide) of the H-2K$^b$ gene. The oligonucleotide introduces an amber stop codon at residue 18 of the signal peptide and the second oligonucleotide results in the insertion of two additional basepairs (GG) between residues 13 and 14 of the signal peptide, thus putting the remainder of the coding sequence out of frame and generating a stop codon at nucleotides 77-79. Nucleotide 1 is defined as the first nucleotide of the coding sequence, i.e. the A of the initiating ATG codon. An additional base change was made in the amber mutant so as to link the creation of a BssHII site to the introduction of the amber mutation. The frameshift mutation (introduction of GG) creates a novel NaeI site. The sequence of the oligonucleotides is shown in Fig. 1. The oligonucleotides were synthesized using a Beckman system 1 DNA synthesizer and purified by high pressure liquid chromatography. The purity of the oligonucleotides was assessed by determining:

a) that $^{32}$P end labelled material gave a single band after denaturing PAGE

b) that each oligonucleotide gave specific priming in M13 dideoxy sequencing

c) that the nucleotide sequence was correct using Maxam and Gilbert procedures modified for oligonucleotides (18).

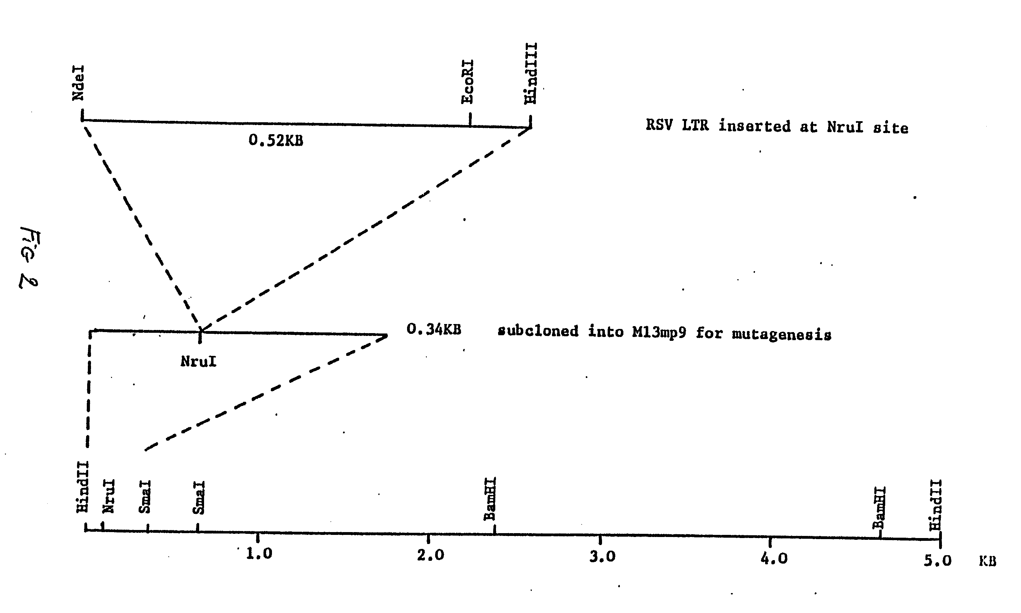

The gapped duplex method (11, 12) was chosen for site specific mutagenesis because it incorporated several refinements of the basic approach using single stranded phage vectors: (i) a brief repair/ligation reaction (ii) the use of a mutant E. coli host to reduce mismatch correction and (iii) the use of a genetic marker (an amber mutation in the plus strand) which allows selection of phage derived from the minus (putatively mutant) strand. A 340bp HindII/SmaI fragment from the 5′ region of the H-2K$^b$ gene was subcloned into M13mp9 to provide a template for the mutagenesis (Fig. 2). Details of the construction of the gapped duplex and the synthesis of the mutants are given above. Those clones containing an NaeI site were isolated using this procedure. DNA sequencing confirmed that these clones contained the expected frameshift mutation. Four clones were obtained which contained an additional BssHII site. However, DNA sequencing revealed that all four positive BssHII clones contained an identical 76bp duplication, beginning precisely at the 5′ end of the mutagenic oligonucleotide. It is possible that this duplication was generated by sequence directed, in vivo nick translation and subsequent ligation. The standard approach involving all-round synthesis and agarose gel purification of the CCC molecules (13) was therefore used to prepare the amber mutant. Clones containing the amber mutation were identified by selective hybridization to the mutagenic oligonucleotide. Eleven putative positives were analyzed by BssHII digestion and by primer extension. The predicted fragment was generated from nine of these clones and original duplication was contained in two, implying the occurrence of some nick translation in vitro.

A single mutant of each type was selected and used to construct complete H-2K$^b$ genes containing the amber and frameshift mutations, respectively.

## Replacement of the endogenous H-2K^b gene promoter with the promoter of the RSV LTR

The RSV LTR has been shown to contain a promoter element that functions particularly efficiently in a variety of eukaryotic cells (20). A 0.5kb NdeI-HindIII fragment containing the RSV promoter was isolated from the plasmid pRSVcat (20). The ends of this fragment were filled in using DNA polymeraseI Klenow fragment and the resulting blunt-ended fragment was inserted into the unique NruI site of both the amber and frameshift derivatives. The EcoRI site in the RSV promoter was used to establish its correct orientation with respect to the gene and this was confirmed by dideoxy sequencing using the mutagenic oligonucleotides as primers.

## Reconstruction of the mutant genes

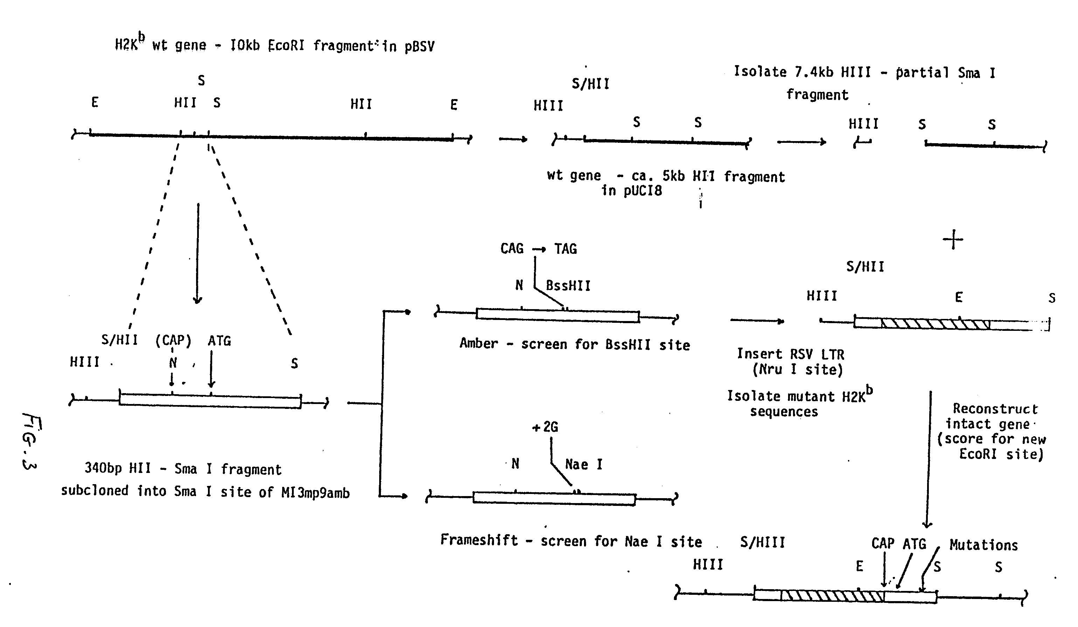

The reconstruction of the mutant genes is summarized in Fig. 3. The 0.8kb fragment carrying each mutant gene segment was isolated from M13mp9 by digestion with HindIII and SmaI and purification from a 1.2% agarose gel. The remaining major part of the gene was isolated from a plasmid containing the wild-type H-2K $^b$ gene cloned in the HindII site of pUC18 (21) as a 7.4kb HindIII/SmaI partial fragment. This fragment, which was separated from the two other closely migrating fragments on a 0.5% low melting point agarose gel, contains the majority of the H-2K$^b$ gene (everything downstream of the 5′ SmaI site) linked to pUC18. Ligation of this 7.4kb fragment to the 0.8kb HindIII/SmaI mutant fragments were ligated together and the ligation products treated with NruI to remove any contaminating full length puC18-H-2K $^b$ sequences, prior to transformation into E. coli. (The mutant gene is resistant to digestion with NruI because the site has been destroyed during insertion of the RSV promoter). Transformants containing the complete mutant genes were identified by the presence of an additional EcoRI site (in the RSV LTR).

The mutant genes were compared with the wild-type H-2K$^b$gene using a panel of restriction enzymes and in each case, the expected pattern of fragments was observed.

Further confirmation that the reconstructed genes contained the expected sequences was obtained by sequencing from the RSV cap site, through the amber or frameshift mutation using the procedures of Maxam and Gilbert (18).

## Construction of the missense gene

The missense gene was constructed by the synthesis of two oligonucleotides with the double stranded DNA sequence

```
5′ CGAATCGCCGACAGGGTCGACGGTACCA   3′
3′ GCTTAGCGGCTGTCCCAGCTGCCATGGTAGGT 5′
```

This sequence contains two differences in the coding sequence of the normal H-2K$^b$ gene, one at the translation initiation site (changing the ATG codon), and one neutral change in the fourth codon to create an out of frame initiation codon. The oligonucleotide was cloned into the normal RSV-H-2K$^b$ gene after partial ApaLI-NruI digestion of the parent plasmid.

## Construction of the gene containing a partial duplication

The partial duplication of the H-2K$^b$ gene was constructed by the isolation of a HindIII-ApaI restriction fragment which contains the first four exons (+ introns) of the H-2 gene. This fragment was ligated to the ApaI site in the second exon to create a partially duplicated gene which generates a frameshifted protein product.

## Functional analysis of the mutant H-2K$^b$ genes in mammalian cells

### 1) Reversion of the frameshift mutation.

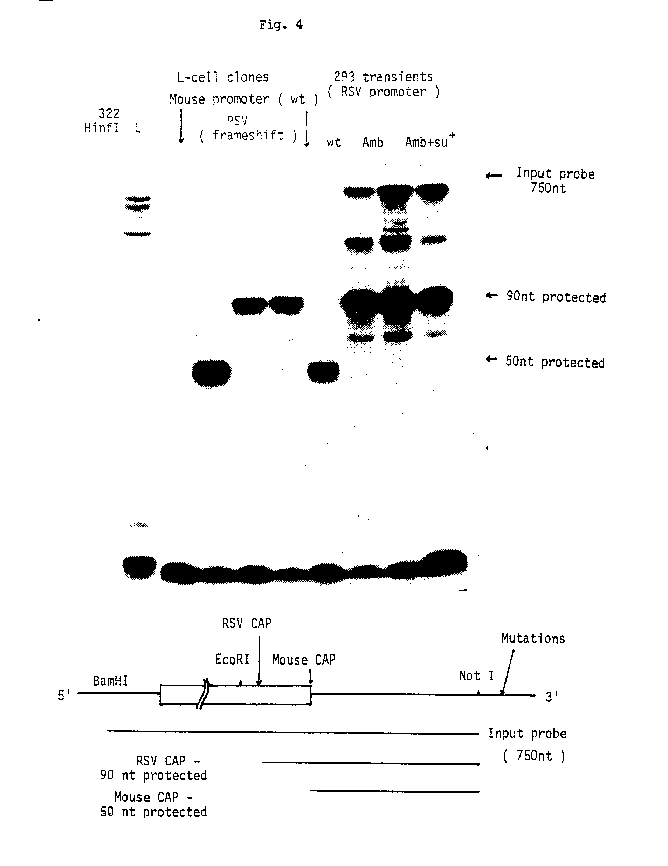

The frameshift gene, cloned in pUC18, has been introduced into mouse L-cells by co-transformation with the HSV thymidine kinase gene of pRT (22). RNA prepared from HAT resistant clones was analysed by SI protection analysis using a probe specific for the 5′ end of the gene (Fig. 4). All clones analysed produced significant quantities of mutant H-2K$^b$ RNA initiated within the RSV LTR. The probe appears to be specific for transcripts from the introduced gene, since no signal was observed in RNA from non transfected L-cells. In contrast to L-cell clones containing the wild-type H-2K$^b$ gene, no H-2K$^b$ protein could be detected on the surface of these clones either using the FACS or the in situ assay described below.

Clone A3 expresses the highest level of mutant mRNA and was selected for preliminary mutagenesis experiments. Two compounds ICR191 and ICR170 (23) which have been found to act as frameshift mutagens in the Ames test (24) and as potent mutagens in mammalian cells (25, 26) are used to attempt to revert the frameshift mutation and isolate cells expressing the wild-type gene. The toxicity of these compounds to L-cells is currently being assessed. A mutagen concentration which allows 10% cell survival is used to treat approximately 10$^7$ A3 cells. The treated cells are allowed to recover and fix the mutation and then divide so as to amplify any revertant cells. The population is then be screened (a) directly using the in situ immunoenzymatic assay described below and (b) by labelling cells with fluorescent antibody, sorting the fluorescent cells using the FACS (27) and continuing this procedure until a highly enriched positive cell population is obtained.

## 2) Suppression of the amber mutation.

Genetic suppression of amber mutations in vivo and in vitro has been demonstrated using an in vitro mutagenised Xenopus leavis tyrosine tRNA gene in an SV40 vector (28). This su+ tRNA gene was isolated from pSV-tT-2(Su+) as a HindIII fragment and inserted upstream of the H-2K$^b$ amber gene, in p228 (29), at the unique HindIII site of the vector. L-cells were cotransfected with this construct, together with a plasmid containing an amber mutant of the cat gene, pRSVcat $^{amb38}$ (30). Transient assays for suppression were carried out two days after transfection, but no H-2K$^b$ protein was detectable using the in situ assay or the FACS, although some suppression (5-15%) of the chloramphenicol acetyl transferase amber gene could be detected. Only about 5% of the cell population appears positive for H-2K$^b$ in a transient assay after transfection with the wild-type gene.

Stable clones, transfected with the amber gene, the suppressor tRNA and pRSVcat$^{amb38}$ and selected for resistance to hygromycin, have now been obtained. H-2K$^b$was not detectable on the surface of any of these clones using the in situ assay. However, there was no suppression of cotransfected pRSVcat$^{amb38}$ amber gene in these clones either. This could reflect the fact that these clones did not receive a copy of the chloramphenicol acetyl transferase amber gene (which can be checked by Southern blotting), or that they do not contain a functional suppressor gene. A further possibility is that a gln tyr substitution at position 18 in the H-2K$^b$ signal peptide might interfere with the targeting of the protein to the cell membrane. Other clones suppressor tRNAs could be tested.

## 3) Reversion of the amber mutation.

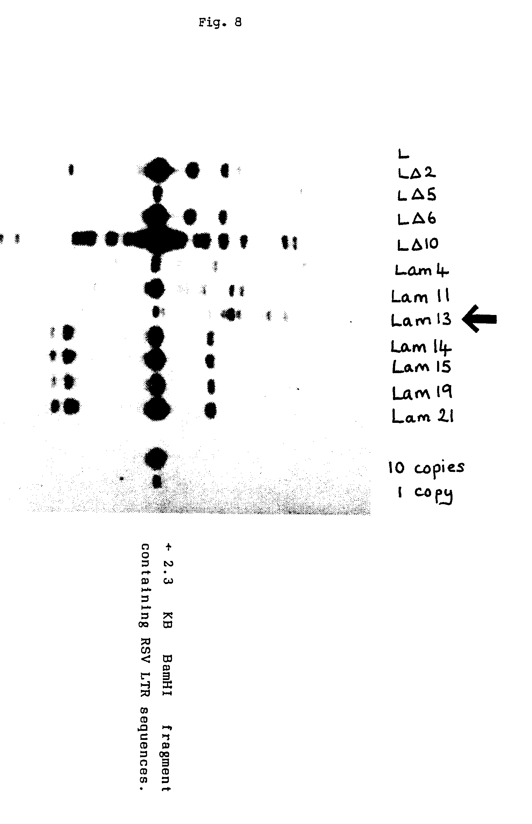

The amber mutant gene cloned in the vector pUC19 was introduced into mouse Ltk⁻ cells by co-transfection with the HSV tk gene in the vector pRT (22). tk+ clones were selected in HAT medium and screened for expression of the H-2K$^b$ gene by SI protection analysis of mRNA using the 5′ H-2K$^b$ probe shown in Figure 4. Clones expressing the highest levels of H-2K$^b$ specific mRNA were chosen for mutagenesis. Southern blots were then carried out on these clones to determine the copy number of the integrated genes using a 500bp NdeIHindIII fragment, from pRSV cat (32), containing RSV LTR sequences, as a probe. Copy number was estimated by comparison with hybridization to known amounts of the input plasmid DNA (Fig. 8). The stability of the L cell clones was asessed by reisolation of RNA followed by SI protection analysis. All clones used in this study were stable for at least four months when grown in HAT medium.

Clones were also tested for suppression of the amber mutation by transfection with the tyrosine suppressor tRNA from Xenopus leavis (see above). All clones which produced H-2K$^b$ specific mRNA were able to produce H-2K$^b$at the cell surface after introduction of the suppressor +RNA gene in a transient assay.

Clone Lam13 was selected for mutagenesis because it contained a single unrearranged copy of the integrated amber H-2K$^b$ gene (estimated visually from Southern blots, Figure 8). Since a single copy of the amber gene produces sufficient mRNA for detectable tRNA suppression, reversion of this gene should also result in detectable levels of protein at the cell surface. Between $5 \times 10^7$ and $2 \times 10^8$ Lam13 cells were exposed to 254nm UV light as described under Materials and Methods. After expansion of the surviving cells for 4-5 days in culture the population was analysed for the presence of revertants, both before and after enrichment of the H-2K$^b$ expressing cells using the EPICS V cell sorter. For sorting, cells were labelled with MV3 (anti H-2K$^b$) followed by an FITC conjugated goat anti-mouse IgG. Propidium iodine was added just prior to sorting to stain dead cells which can then be gated out on the basis of high red fluorescence. The top 3% of the cells exhibiting the highest green fluorescence were collected and grown for 3-4 days in HAT medium before analysis using the in situ immunoenzymatic assay. At the same time as cells were prepared for sorting, $2 \times 10^6$ cells were plated onto a 10cm dish and grown for three days, then analysed in situ.

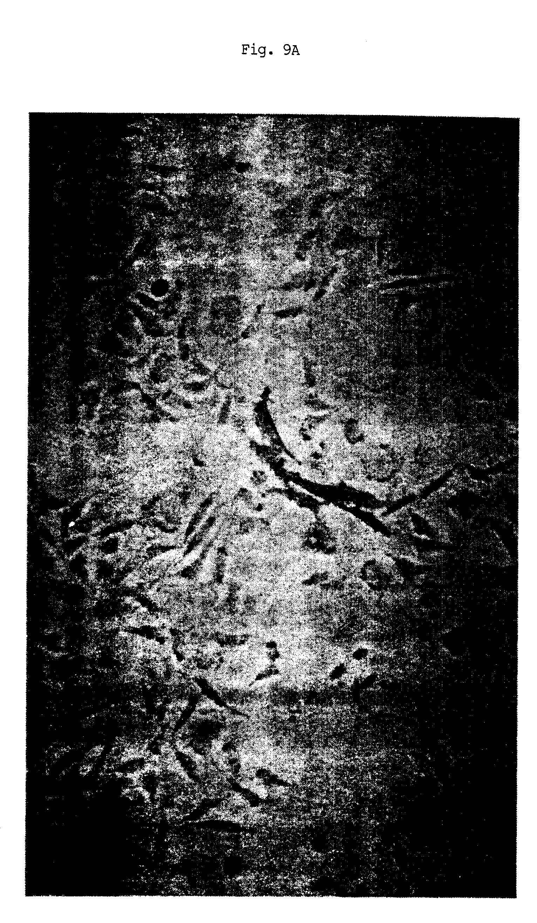

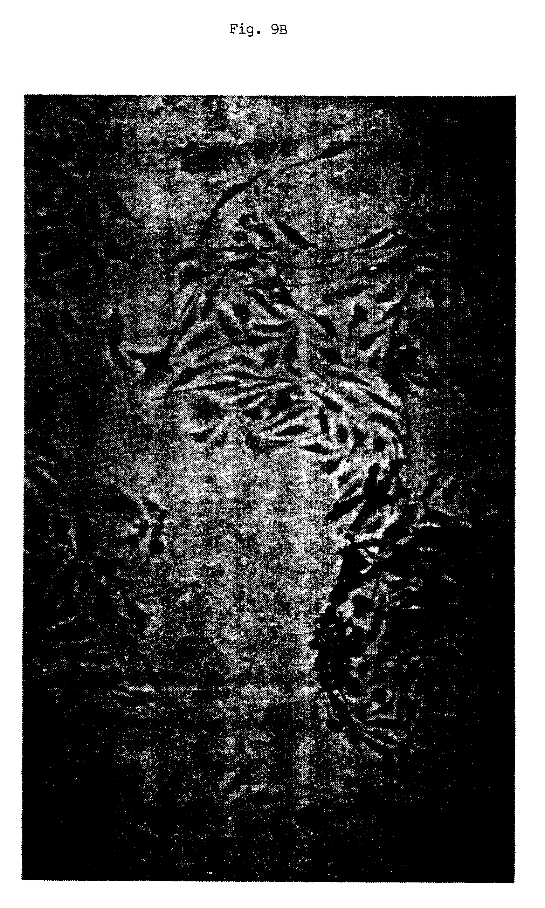

Clusters of red/brown staining cells representing putative revertants were found in three independent experiments, both prior to and after sorting (Figure 9). Mutated populations of Lam13 cells were subjected to repeated cycles of sorting in order to enrich the revertant population. Individual revertants were cloned and the nature of the mutations which occurred by DNA sequencing were analyzed. The DNA sequence of the reverants confirmed that the Lam13 cells had reverted to the non-mutated DNA sequence.

4) Reversion of the frameshift gene after transfection

Recent preliminary experiments have provided evidence that the frameshift gene is also revertable. Hygromycin resistant A431 colonies transfected with a frameshift EBV construct were stained (two weeks post trans fection) for H-2K$^b$ in situ and, in two independent experiments, colonies containing H-2K$^b$ expressing cells were detected at a frequency of about 1 in 100 colonies. It is believed that the frameshift mutation has been reverted owing to (i) the intrinsic instability of EBV constructs in A431 cells and/or (ii) the high incidence of mutagenesis associated with transfection. Spontaneous reversion has not been observed for stably tranfected cell lines.

## Construction of plasmids containing multiple copies of the H-2K$^b$ gene

In order to facilitate the construction of a plasmid containing multiple copies of the mutant H-2K$^b$ genes in head to tail orientation, each mutant gene was subcloned as a 5.5kb HindII fragment into the HindII site of the vectors pUC18 and pUC19 so as to obtain the gene in both orientations with respect to the sites in the polylinker (the H-2K$^b$ gene is only stable in one orientation with respect to pUC sequences, hence the need to clone it into both pUC18 and pUC19). The mutant genes were then isolated as partial EcoRI-total HindIII fragments from each vector. During isolation from pUC18 the HindIII site was dephosphorylated with CIP (31) and during isolation of the gene from pUC19 the EcoRI site was dephosphorylated. This procedure yielded two 5.5 kb fragments containing the H-2K$^b$ gene, one of which was flanked at its 5' end by a dephosphorylated HindIII terminus, and at its 3' end by a phosphorylated EcoRI terminus and the other which was flanked at its 5' end by a dephosphorylated EcoRI terminus and at its 3' end by a phosphorylated HindIII terminus.

The two fragments were ligated together in equal quantities at high DNA concentration. (The necessity for a dephosphorylated HindIII or EcoRI terminus to pair with a complementary phosphorylated terminus should increase the probability of formation of multimers containing the genes arranged in head to tail orientation.) The ligation products were phosphorylated with T4 polynucleotide kinase to replace the terminal phosphates and the protruding termini were then blunted using DNA polymerase I Klenow fragment. These products were then ligated to the modified p228 cosmid vector, pSCM100, (described below) which had been cleaved at the ClaI site and the ends blunted with Klenow.

The products of the ligation were packaged and used to infect E. coli ED8767 (22). The first attempt at this procedure gave a poor yield of cosmid clones, a total of 11, most of which contained deletions, but digestion of one of the clones with SalI indicated that it contains three copies of the H-2K$^b$ gene.

In addition to the construction of pSCM100 cosmids containing multiple copies of the mutant genes, a similar construct may be prepared using the wild-type gene. This may be used in studies on plasmid copy number, stability and gene expression in various human cell lines. This allows useful controls to be performed in which the level of message is correlated with level of protein at the cell surface.

## Modification of the vector

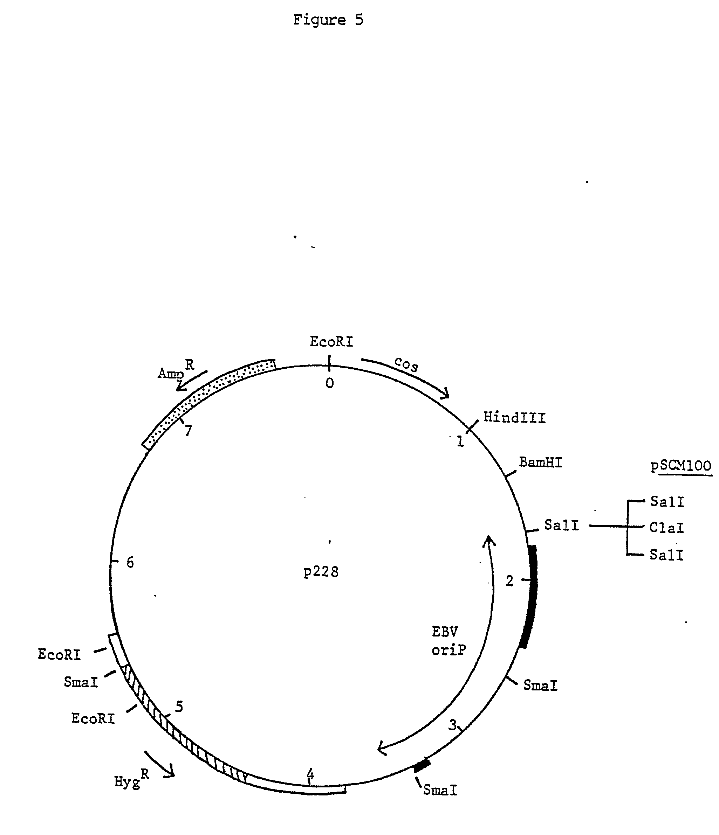

In order that the multimer of the H-2K$^b$ gene could be removed from the vector as a single fragment, the chosen vector, p228 (29) (Fig. 5) was modified at its single SalI site. The plasmid was cleaved with SalI and the protruding ends were filled with DNA polymeraseI Klenow fragment. ClaI linkers, d(pCATCGATC), were then ligated to the resulting blunt ends of the plasmid. The ligation products were digested with ClaI and religated. The ligation of the terminal cytosine of the ClaI linker to the filled in SalI site restores the SalI site on both sides of the linker. This plasmid, designated pSCM100, now contains the sequence

GTCGACATCGATGTCGAC

SalI     ClaI     SalI

in place of the original sequence

GTCGAC

SalI

This new vector was cleaved with ClaI and the ends filled in before ligation to:
  a) a single copy of the wild-type H-2K$^b$ gene linked to its own promoter.
  b) a single copy of the H-2K$^b$ gene under the control of the RSV promoter.
  c) the RSV-H-2K$^b$ gene multimers.
Similar constructs were prepared for the two mutant gene.


Expression of the H-2K $^b$ gene in human cell lines

Some preliminary investigations were made into the suitability of using the human Epstein Barr virus transformed B lymphoblastoid cell line 262 as a tester line. This line contains a deletion in the globin gene region. The presence of the Epstein Barr virus genome would make it permissive for the replication of the oriP vector, p228. However, chloramphenicol acetyl transferase assays indicated that this line was transfected very inefficiently by a variety of procedures (Calcium phosphate co-precipitation, electroporesis and DEAE dextran). A more significant disadvantage of 262 is that it grows in clumps in suspension and several attempts to isolate a variant which either sticks to the substratum or grows as a single cell suspension were unsuccessful.

Three human cell lines were obtained from Oncogene Sciences, Inc.:
  A431 = an epidermal carcinoma of vulva
  A549 = a rhabdomyosarcoma
  A673 = a lung carcinoma

In order to determine the suitability of these cell lines as tester lines, the following had to be established:
  1) The presence of a transformation system suitable for introducing foreign DNA into the cell line.
  2) That the H-2K $^b$ gene is efficiently expressed and that a protein is detectable at the cell surface.


3) That multiple copies of the H-2K$^b$ gene can be stably maintained in the cell line.

The calcium phosphate/DNA coprecipitation procedure (14) was used successfully to transform all three cell lines. Optimum conditions for transformation were assessed by introducing the plasmid pRSVcat (20) into each cell line and assaying for chloramphenicol acetyl transferase activity (20). Exposure of the cells to the CaPO$_4$/DNA coprecipitate for four hours followed by a one minute glycerol shock (32) was found to give optimal chloramphenicol acetyl transferase activity in A431 and A673 cells, whereas chloramphenicol acetyl transferase activity in A549 cells was increased by extending the period of the glycerol shock to five minutes.

These conditions were therefore used to introduce the wild-type H-2K$^b$ gene (a) under the control of its own promoter and (b) under the RSV promoter, cloned in pSCM100, into each cell line. Stable transformants were selected for resistance to the antibiotic hygromycin (33) (150 micrograms/ml for A431 and A673 and 300 micrograms/ml for A549). The frequency of stable transformants was in the order of $2-5 \times 10^{-5}$ for A431 and A673 and between $10^{-5}$ and $10^{-6}$ for A549. In a preliminary experiment, the total population of hygromycin resistant cells from each transformation of A431 and A673 was pooled (50-100 clones) and labelled with the anti-H-2K$^b$ antibody HB41, followed by FITC-GAM Ig and analyzed using a fluorescence activated cell sorter (BD FACS II). In each case approximately 10-20% of the cells were expressing H-2K$^b$ at the cell surface.

Individual clones were picked from similar transformation and analyzed for expression of H-2K$^b$ by radio-immune binding. Five positive A673 clones and five positive A431 clones were identified which contained H-2K$^b$ under the control of the RSV promoter and another three positive A673 end two A431 clones which contained H-2K$^b$ under the control of the endogenous promoter. These clones expressed varying amounts of H-2K$^b$ and the highest expressers bound a similar amount of antibody to an L-cell clone transfected with H-2K$^b$ and selected for high level expression (Table 1).

## TABLE 1

| Clone[b) | | Transfected gene H-2K$^b$ | RSV/H-2K$^b$ | cpm $^{125}$I bound[a) |
|---|---|:---:|:---:|---:|
| A431 | A2 | + | | 1838 |
| A431 | A4 | + | | 950 |
| A431 | B2 | | + | 2553 |
| A431 | B3 | | + | 1238 |
| A431 | B6 | | + | 1163 |
| A431 | B7 | | + | 5399 |
| A431 | B8 | | + | 4694 |
| A673 | A21 | + | | 4878 |
| A673 | A22 | + | | 5783 |
| A673 | B2 | | + | 3055 |
| A673 | B4 | | + | 1740 |
| A673 | B8 | | + | 3442 |
| Ltk⁻ | C4 | + | | 4129 |
| | | | | |
| A431 | | – | – | 533 |
| A673 | | – | – | 186 |
| Ltk⁻ | | – | – | 154 |

a) cpm $^{125}$I bound in an indirect radiobinding assay. $10^6$ cells were incubated with HB41 (α-H-2K$^b$) supernatant, washed, then incubated with $^{125}$I labelled rat α-mouse kappa light chain (187.1). The results are the mean of two replicates.

Table 1: Continued

b) Only those clones identified in one screening experiment are shown. Three additional A673 H-2k$^b$ positive clones and one A549 clones were identified in separate screening experiments (data not shown).

A single A549 clone containing the H-2K$^b$ gene expressed from its endogenous promoter was obtained from these experiments.

The DNA/CaPO$_4$ coprecipitates used in this experiment also contained the plasmid pSVOB-H2.9 containing the EBNA-1 gene (7) in a 10-fold excess over the pSCM100 (H-2K$^b$) plasmids. If these clones contain a functional integration of pSVOB-H2.9, the pSCM100 vector should replicate autonomously. Once it is established, after analysis of the clones, whether or not the plasmid can be recovered from a Hirt supernatant (34), the copy number of the plasmid in different cell lines will be determined. This number can be compared with the copy number of a plasmid containing multiple copies of the H-2K$^b$ gene in order to determine which system maintains the maximum total number of copies of the H-2K$^b$ gene.

As an alternative approach to obtaining cells capable of supporting the replication of oriP containing plasmids, the three cell lines were transfected with the plasmid pSVOB-H2.9 which contains the EBNA-1 gene. Transformants were selected in G418 containing medium. These clones can then be transfected with the pSCM100/H-2K $^b$ plasmids.

As an alternative approach to obtaining cells capable of supporting the replication of oriP containing plasmids, the three cell lines were transfected with the plasmid pSVOB-H2.9 which contains the EBNA-1 gene. Transformants were selected in G418 containing medium. These clones can then be transfected with the pSCM100/H-2K$^b$ plasmids.

The observation that H-2K$^b$ is expressed in all three cell lines and that high levels of protein are detectable on the cell surface makes all these lines suitable candidates for a tester line. The clones may be suitable in an _in situ_ immunoenzymatic test.

### Use of vectors containing both OriP and EBNAI sequences

Studies on the stability of the A431 clones containing the wild H-2K$^b$ gene cloned in pSCM100 revealed that these clones are not stable. Analysis of clones B2, B6, and B7 (Table 1) two months after cloning revealed that these clones contained many cells not expressing H-2K$^b$ (B2 approximately 10% expressing H-2K$^b$ , B6 approximately 30%, and B7 approximately 50%). The H-2K$^b$ expressing cells from these populations were collected using the FACS II cell sorter, grown and analyzed and were again found to contain a mixture of H-2K$^b$ expressing and non-expressing cells. Preliminary data suggested that these clones contained episomes one month after isolation of clones. Analysis after a further two months resulted in the recovery of very small quantities of episomes (less than one copy per cell) which had apparently undergone deletion.

One clone, B8, which remained approximately 90% positive was found to contain sequences hybridizing to the H-2K$^b$ construct integrated in the genomic DNA.

A possible explanation for this instability was the cotransformation strategy used to obtain these clones. Possibly the pSVOB-H2.9 and the pSCM100 (H-2K$^b$ ) plasmids could have recombined during transfection resulting in this instability.

An alternative strategy was then followed: A431 cells were first transfected with pSVOB-H2.9 and G418 resistant clones isolated. These transformants were then transfected with pSCM100 containing the H-2K$^b$ gene.

Transfection efficiencies of these cells were still very poor, suggesting that these constructs did not replicate efficiently in A431 when containing pSVOB-H2.9. Subsequent Southern blot analysis of some of these clones revealed that they contained H-2K$^b$ sequences integrated into the genome.

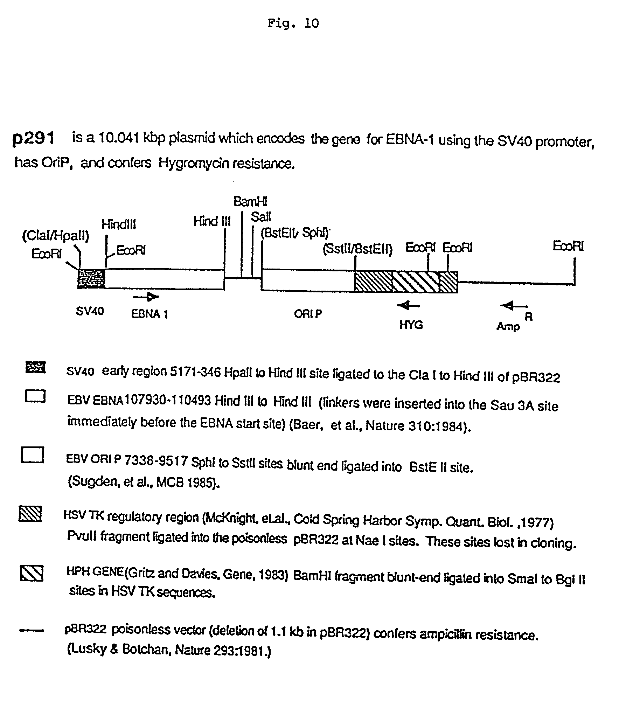

It seemed possible that the A431 cell lines transfected with pSVOB-H2.9 were not producing sufficient EBNA I protein to allow replication of the OriP constructs in these cells. We therefore decided to use vectors containing both EBNA I and OriP. For this we chose the vector p292 (Sugden, unpublished, Figure 10). The H-2K$^b$ wild-type and mutant genes were cloned in the SalI site (Figure 10). These constructs were introduced into A431 cells by CaPO$_4$ co-precipitation. Transfection frequency was very high (approximately $1.5 \times 10^3$ colonies/10 micrograms DNA/$10^6$ cells), indicating that these constructs appeared to replicate in A431 cells. 10-12 individual clones were isolated from all transfections and analyzed for production of episomes and H-2K$^b$ specific RNA (and protein in the case of the wild-type gene). Many of the clones analyzed contained episomal DNA but in every case the DNA had been rearranged with respect to the input DNA. This rearrangement was not dependent on the orientation in which the H-2K$^b$ gene was cloned in the vector.

A further variable was then tested by changing the recipient cell line. HeLa is an adherent cell line and therefore suitable for _in situ_ immunoenzymatic staining. The same p292 constructs containing the H-2K$^b$ cloned in the SalI site were introduced into HeLa and were found to replicate in a stable manner. In addition, the copy numbers were higher (10-50 copies/cell) than in A431 cells (Figure 12). Clones containing the wild-type H-2K$^b$ gene were found to be 100% H-2K$^b$ positive by antibody staining.

We have therefore introduced all the mutant constructs into HeLa cells and analyzed the tranfected cells. After four months of continuaous culture, the episomes remained unrearranged and are still present at 10-50 copies/cell.

## Development of a detection assay

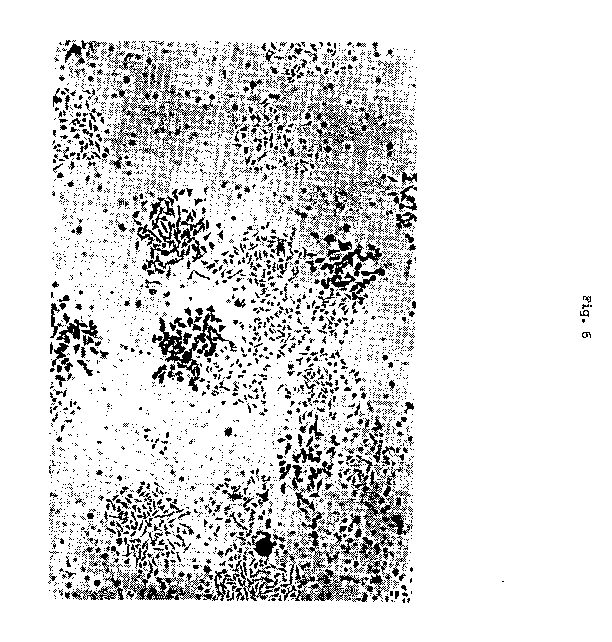

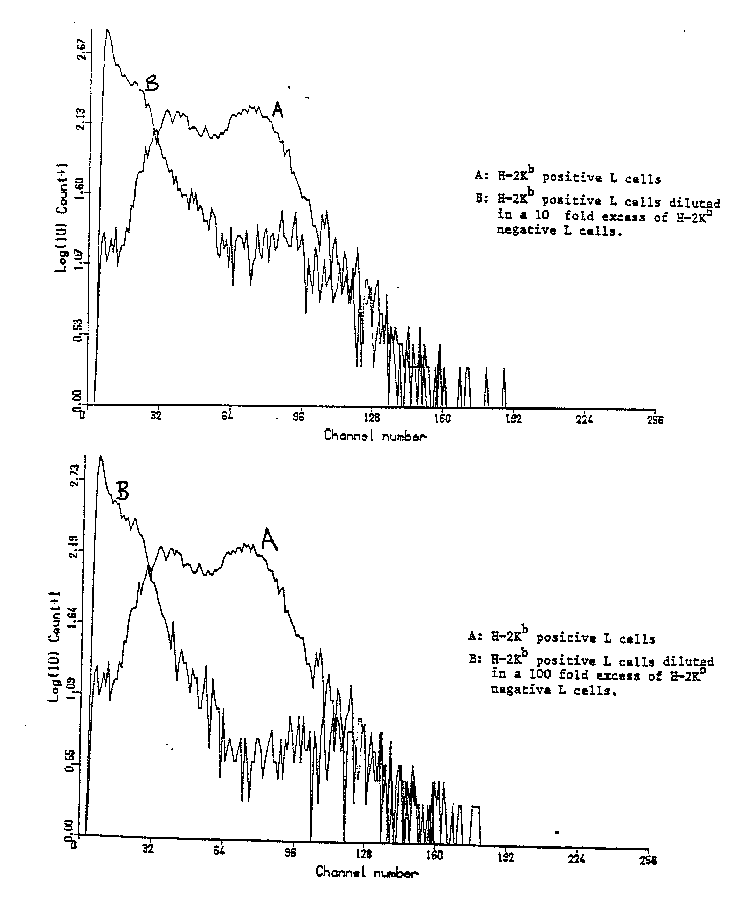

By using the immunoenzymatic in situ assay described in the methods section, one is able to detect single cells expressing the H-2K$^b$antigen which stains red-brown, this color clearly distinguishing them from non-expressing cells which appear colorless (Fig. 6). When colonies (10-20 cells in size) are stained they can be seen with the naked eye. Fig. 7 shows FACS profiles of mixtures of L cells positive and negative for expression of H-2K$^b$. $10^6$ cells were incubated with HB41 hybridoma supernatant (anti-H-2K$^b$), washed and incubated with FITC-goat anti-mouse IgM (A: H-2K$^b$ positive L cells, B: H-2K$^b$ positive L cells diluted in a ten fold excess of H-2K$^b$ negative L cells, C: H-2K$^b$ positive L cells, and D: H-2K$^b$ positive L cells diluted in a one hundred fold excess of H-2K$^b$ negative L cells). In Fig. 7, L-cells expressing H-2K$^b$ were mixed with a ten fold excess of non-expressing L-cells, plated and grown for 7 days to allow the formation of colonies.

The in situ assay was then performed on these cells, as described in the methods section. Using black and white photography the positive cells appear dark in comparison with the unstained negative cells. The actual color of the positive cells in red-brown when 3-aminoethylcarbazole is used as a substrate for horseradish peroxidase. This gives a good contrast with unstained negative cells using inverted light microscopy.

Substrates other than 3-aminoethylcarbazole have also been tested with horseradish peroxidase conjugates (i.e. O-dianisidine, 4-chloronaphthol) but their colored reaction products (orange and violet-black, respectively) did not give a contrast with non-stained cells as clear as that given by 3-aminoethylcarbazole using ordinary inverted microscopy.

Alkaline phosphatase-antibody conjugates were also tested without any improvement on the 3-aminoethylcarbazole-horseradish peroxidase procedure.

The ability to detect single cells with this procedure is the main advantage of the in situ assay in comparison with analysis of populations using cytofluorography. For instance, we have observed in reconstruction experiments in which cells positive and negative for the H-2K$^b$ antigen were mixed in various ratios, that cell populations in which the positive fraction was equal or less than $10^{-2}$ gave no signal by cytofluorography (Fig. 7). In contrast, positive cells were found using the immunoenzymatic in situ assay in populations containing $10^{-5}$ positive cells. At present, the main disadvantage of this method is the operator effort required to screen populations contain ing low frequency of positive cells. Thus, any attempt at automation is encouraged. Because of this a systematic scoring of populations containing positive and negative cells has not yet been performed at frequencies below $10^{-5}$ positive cells.

For populations more highly enriched in positive cells ($30^{-1}$, $900^{-1}$) a very good correlation was found between the number of stained cells counted and the known proportion of positive cells in the population. Thus, populations with a three-fold serially diluted content of positive cells gave a three-fold decreased number of stained cells. With mouse L-cells expressing the transfected H-2K$^b$ antigen, this in situ method does not seem to show any non-specific background staining problem. Using a replica plating method we were able to get copies of colonies growing in a plate seeded with a mixed population of negative and positive cells. After identification of positive colonies by staining, we went back to the replica plate and isolated those putative positive colonies. These colonies were subsequently analyzed by cytofluorography with the result that stained colonies always gave positive signals, whereas non-stained colonies failed to give any signal.

## Production of anti H-2K$^b$ antibodies

First attempts to produce anti-H-2K$^b$ antibodies were made following an immunization protocol in which B10-A(4R) mice (K$^k$, I-A$^k$, I-E$^b$, D $^b$ haplotype) were injected with B10 mouse splenocytes (d haplotype) looking for a very specific response. This protocol did not give a good response in terms of antibody production as determined by binding to EL-4 cells. However, one hybridoma constructed in this way is included in the following analysis.

The immunization protocol was changed and Balb/c mice (d haplotype) were used as recipients of Balb/b mouse splenocytes (b haplotype). Serum antibody titres were now higher and accordingly, a higher frequency of positive hybridoma cultures was obtained. (15 out of 560 cultures screened, which is in the range of the known low efficiency of anti-MHC monoclonal antibodies produced in allogeneic immunizations (35).

Because of the nature of the immunization protocol which could raise antibodies against all MHC region products, it was necessary to use a highly specific screening assay. This was done by suing a mouse fibroblast cell (L-cells) transfected with the cloned H-2K$^b$gene as target cells in radiobinding experiments. Antibodies to class I MHC products (mainly K and/or D) can be identified and information about their anti-H-2K$^b$ specificity can be provided, by comparison with results obtained in parallel with a non H-2K$^b$ expressing L-cell clone.

Seven cultures remained positive after initial expansion and cloning and they were tested in the in situ assay as described above. Human cell lines (A673 and A549) transfected with the cloned H-2K$^b$ gene were used instead of mouse L-cells.

Experiments showed that staining in the in situ test did not correlate with the binding data to H-2K$^b$ bearing L cell clones. Thus antibodies showing the strongest activity in the radiobinding assay did not give any staining, and no staining was seen with the ATCC antibody HB41 which was being used as a positive control.

At least one of the monoclonal antibodies gives a good signal in the in situ assay using human cell lines. The protocol involves increasing the concentration of the second antibody and in a refinement the second antibody was replaced by biotinylated goat anti mouse Ig and this was then bound to streptavidin coupled to horseradish peroxidase. The staining of certain of the monoclonal antibodies seen in the experiments described has been shown to be non-specific.

This hybridoma line has been further characterized. It makes a high affinity anti-H-2K$^b$ antibody (HV3). It is an IgG antibody which immunoprecipitates H-2K$^b$ from cell lysates (Figure 12).

The hybridoma grows well as an ascitic tumour and ascites fluid with a very high antibody titer ($10^{-5}$) has been obtained. Figure 13 shows the chromatographic profile obtained in a hydroxyapatite column used for isolation of MV3 from ascites fluid. It also binds to protein A-sepharose which provides a useful alternative isolation procedure.

When analysed by radiobinding to a panel of splenocytes from most of the common mouse haplotypes, it appears to be specific for the H-2K$^b$ haplotype (Table II) indicating that it is very likely recognizing a polymorphic determinant on the H-2K$^b$ molecule.

## TABLE II

| Mouse strains | Haplotype | Antibody bound (cpm) |
|---|---|---|
| B10 | b | 11464 |
| Balb/c | d | 511 |
| B10.RIII | r | 467 |
| B10.S | s | 552 |
| B10.M | f | 118 |
| C3H/N3 | p | 206 |
| DBA/1 | g | 151 |
| C3W/HE | k | 470 |
| B10.A(4R) | ($K^kD^b$) | 602 |

Specificity of anti-H-2K$^b$ monoclonal antibody MV3. It was determined in a direct radiobinding assoay using $5 \times 10^5$ splenocytes and $10^5$ cpm of $^{125}$I-MV3 in duplicate. Non-specific binding (cpm bound to B10 splenocytes in the presence of an excess of non-labelled MV3 antibody, 530 cpm, was subtracted).

References

1. Bartsch, H., et al., (1980) molecular and Cellular Aspects of Carcinogen Screening tests. montesano, R., Bartsch, H. and Tomatis, L. (Eds.) IARC Scientific Publications - International Agency for Research on Cancer, Lyon, vol. 27, p. 179.

2. Carrano, A.V., et al., (1978) Nature, vol. 271, p. 551.

3. Ames, B.N., et al., (1975) Mutation Research, vol. 31, p. 347.

4. Rinkus, S.J., et al., (1979) Cancer Research, vol. 39, p. 3289.

5. Weiss, E., et al., EMBO J., vol 2, p. 453.

6. Ozato, K., et al., J. Immunol., vol. 126, p. 317.

7. Yates, J.L., et al., (1985) Nature, vol. 313, p. 812.

8. Young, R., et al., (1983) Proc. Natl. Acad. Sci. U.S.A., vol. 80, p. 1194.

9. Litman, D.R., et al., (1985) Cell, vol. 40, p. 237.

10. Holtkamp, B., et al., (1981) Nature, vol. 289, p. 66.

11. Kramer, W., et al., (1984) Nucl. Acids. Res., vol. 12, p. 9441.

12. Kramer, B., et al., (1984) Cell, vol. 38, p. 879.

13. Zoller, M.J., et al., (1983) Methods in Enzymology, vol. 100, p. 468.

14. Wigler, M., et al., (1979) Cell, vol. 16, p. 777.

15. Neumann, E., et al., (1982) EMBO J., vol. 1, p. 841.

16. Sompayrac, L.M., et al., (1981) Proc. Natl. Acad. Sci. USA., vol. 78, p. 7575.

17. Berk, A.J., et al., (1977) Cell , vol. 12, p. 721.

18. Maxam, A.M., et al., Methods in Enzymology, vol. 65, p. 499.

19. Kohler, G. et al., (1975) Nature, vol. 256, p. 495.

20. Gorman, C.M., et al., (1982) Proc. Natl. Acad. Sci. U.S.A., vol. 79, p. 6777.

21. Norrander, J., et al., (1983) Gene, vol. 26, p. 101.

22. Grosveld, F.G., et al., (1982) Nucl. Acids. Res., vol. 10, p. 6715.

23. Ames, B.N. et al., (1966) Cold Spring Harbor Quant. Symp. Biol., vol. 31, p. 221.

24. DeFlora, S., et al., (1984) Mutation Res. , vol. 133, p. 161.

25. O'Neill, J.P., et al., (1978) Cancer Res., vol. 38, p. 596.

26. Fuscoe, J.C., et al., (1982) Mutation Res., vol. 96, p. 15.

27. Berman, J.W., et al., (1984) Proc. Natl. Acad. Sci. U.S.A., vol. 81, p. 7176.

28. Laski, F., et al., (1982) Proc. Natl. Acad. Sci. U.S.A., vol. 79, p. 5813.

29. Sugden, B., unpublished.

30. Burke, J.F., et al., (1985) Nucl. Acids Res., vol. 13, p. 6265.

31. Ullrich, A.J., et al., (1977) Science, vol. 196, p. 1313.

32. Gorman, C.M., et al., (1982) Mol. Cell. Biol., vol. 2, p. 1044.

33. Gritz, L. et al., (1983) Gene, vol. 25, p. 179.

34. Hirt, B. (1967) J. Molec. Biol., vol. 26, p. 365.

35. Lemke, H., et al., (1978) Nature, vol. 271, p. 249.

**Claims**

1. A method for testing a substance to determine whether the substance is mutagenic comprising the following steps:

a) contacting with the substance a mammalian tester cell, the cell comprising DNA carrying a mutation which renders the DNA incapable of directing production of a detectable marker, which DNA is capable of undergoing back mutation so that the detectable marker is produced under conditions which permit back mutation of the DNA;

b) treating the resulting tester cell during a period of time such that cell doubling of the tester cell occurs no more than about three times under conditions which permit production of the detectable marker; and

c) detecting the presence of the detectable marker, such presence indicating that the substance is mutagenic.

2. A method of claim 1, wherein the DNA is foreign DNA.

3. A method of claim 1, wherein the DNA carrying the mutation is present in the tester cell in multiple copies.

4. A method of claim 1, wherein the mammalian tester cell is a mouse cell.

5. A method of claim 1, wherein the mammalian tester cell is a human cell.

6. A method of claim 4, wherein the mouse tester cell is an L cell.

7. A method of claim 1, wherein the mammalian tester cell is a hepatoma cell.

8. A method of claim 1, wherein the mammalian tester cell is an adherent cell.

9. A method of claim 1, wherein the mammalian tester cell is a nonadherent cell.

10. A method of claim 1, wherein the mammalian tester cell is a hypermutagenic cell.

11. A method of claim 1, wherein the mammalian tester cell is deficient in at least one DNA repair enzyme.

12. A method of claim 11, wherein the DNA repair enzyme is an excission repair enzyme.

13. A method of claim 1, wherein the mammalian tester cell is a Xeroderma pigmentosum cell.

14. A method of claim 1, wherein the mammalian tester cell is an Ataxia telangectasia cell.

15. A method of claim 1, wherein the mammalian tester cell is hypersensitive to ultraviolet radiation.

16. A method of claim 1, wherein the mammalian tester cell is hypersensitive to ionizing radiations.

17. A method of claim 1, wherein the mammalian tester cell is hypersensitive to alkylating agents.

18. A method of claim 1, wherein the mammalian tester cell is hypersensitive to cross-linking compounds.

19. A method of claim 1, wherein the mammalian tester cell is hypersensitive to chemical carcinogens.

20. A method of claim 1, wherein the mammalian tester cell has a high frequency of chromosome aberrations.

21. A method of claim 1, wherein the mammalian tester cell has a high frequency of spontaneous sister chromatid exchanges.

22. A method of claim 1, wherein the mammalian tester cell line has an altered nucleotide pool size.

23. A method of claim 1, wherein the contacting is carried out in the presence of metabolic activators.

24. A method of claim 23, wherein the metabolic activators are present in the form of a liver extract.

25. A method of claim 23, wherein the metabolic activators are liver enzymes.

26. A method of claim 1, wherein the DNA additionally encodes a selectable marker for growth of the tester cell.

27. A method of claim 1, wherein the DNA additionally encodes a selectable marker for growth of bacterial cells which comprise the DNA.

28. A method of claim 1, wherein the DNA additionally encodes a selectable marker for growth of the tester cell and for growth of bacterial cells which comprise the DNA.

29. A method of claim 1, wherein the mutation is a mutation in a stop codon.

30. A method of claim 1, wherein the mutation comprises a base substitution in the DNA.

31. A method of claim 1, wherein the mutation comprises a nucleotide insertion in the DNA.

32. A method of claim 1, wherein the mutation comprises a deletion in the DNA.

33. A method of claim 1, wherein the mutation comprises a point mutation in the DNA.

34. A method of claim 1, wherein the mutation comprises a frame-shift mutation in the DNA.

35. A method of claim 1, wherein the mutation comprises a nonsense mutation in the DNA.

36. A method of claim 1, wherein the mutation comprises a missense mutation in the DNA.

37. A method of claim 1, wherein the mutation is a duplication in the DNA.

38. A method of claim 1, wherein the mutation is in a coding sequence of the DNA.

39. A method of claim 1, wherein the mutation is in a regulatory region of the DNA.

40. A method of claim 39, wherein the regulatory region is a promoter sequence.

41. A method of claim 1, wherein the mutation is in an RNA processing sequence.

42. A method of claim 1, wherein the detectable marker is RNA.

43. A method of claim 42, wherein the RNA is mRNA.

44. A method of claim 1, wherein the detectable marker is detected using fluorescence.

45. A method of claim 1, wherein the detectable marker is detected using luminescence.

46. A method of claim 1, wherein the detectable marker is detected using colorimetry.

47. A method of claim 1, wherein the detectable marker is detected using an antibody directed to the marker.

48. A method of claim 1, wherein the detectable marker is a protein.

49. A method of claim 48, wherein the protein is an enzyme.

50. A method of claim 48, wherein the protein is a cell surface protein.

51. A method of claim 1, wherein the detecting comprises visualizing single cells.

52. A method of claim 1, wherein the detecting comprises replica plating.

53. A method of claim 1, wherein the detecting is manual.

54. A method of claim 1, wherein the detecting is automated.

55. A method of claim 54, wherein the automated detection is performed with a fluorescense activated cell sorter.

56. A method for testing a substance to determine whether the substance is mutagenic comprising the following steps:

a) contacting with the substance an mammalian tester cell, the cell comprising foreign DNA carrying a mutation which renders the DNA incapable of directing production of a detectable marker, which DNA is capable of undergoing back mutation so that the detectable marker is produced under conditions which permit back mutation of the DNA;

b) treating the resulting tester cell under conditions which permit production of the detectable marker; and

c) detecting the presence of the detectable marker, such presence indicating that the substance is mutagenic.

57. A method for testing a substance to determine whether the substance is mutagenic comprising the following steps:

a) contacting with the substance a mammalian tester cell, the cell comprising multiple copies of foreign DNA carrying a mutation which renders the DNA incapable of directing production of a detectable marker, which DNA is capable of undergoing back mutation so that the detectable marker is produced under conditions which permit back mutation of the DNA, and which DNA encodes a selectable marker for growth of the tester cell;

b) treating the resulting tester cell under conditions which permit production of the detectable marker; and

c) detecting the presence of the detectable marker, such presence indicating that the substance is mutagenic.

58. A mammalian tester cell comprising foreign DNA carrying a mutation which renders the DNA incapable of directing production of a detectable marker but which is capable of undergoing back mutation so that the detectable marker can be produced.

59. A human tester cell of claim 58.

60. A mammalian tester cell of claim 58, wherein the foreign DNA is mammalian DNA.

61. A mammalian tester cell of claim 58, wherein the foreign DNA is bacterial DNA.

62. A mammalian tester cell of claim 58, wherein the foreign DNA is viral DNA.

63. A mammalian tester cell of claim 58, wherein the foreign DNA is plant DNA.

64. A mammalian tester cell of claim 58, wherein the foreign DNA is fungal DNA.

65. A mammalian tester cell of claim 58, wherein the foreign nucleic acid is RNA.

66. A hypermutagenic, mammalian tester cell of claim 58.

67. A mammalian tester cell of claim 58, comprising multiple copies of the DNA, each copy carrying a mutation which renders the DNA incapable of directing production of a detectable marker but which is capable of undergoing back mutation so that the detectable marker can be produced.

68. A mammalian tester cell of claim 58, wherein the foreign DNA additionally encodes a selectable marker for growth of the tester cell.

69. A mammalian tester cell of claim 58, wherein the foreign DNA additionally encodes a selectable marker for growth of bacterial cells which comprise the DNA.

70. A mammalian tester cell of claim 58, wherein the foreign DNA additionally encodes a selectable marker for growth of the tester cell and for growth of bacterial cells which comprise the DNA.

71. A mammalian tester cell of claim 58, wherein the detectable marker is a cell surface protein.

72. An in vivo mammalian tester cell of claim 58.

Fig. 1

TYR
↑
Suppression       Bss HII

5'      CCG ACT (T)AG AC(G) CGC GCG      3'  Amber mutant Sequence

PRO THR STP THR ARG ALA

3'    A GGC TGA (A)TC TG(C) GCG CGC C  5'  (Amber oligo)

3' ,CGC CGG CGG | CC GAC CGA GGC  5'  (Frameshift oligo)

| 5' | CTG | CTG | TTG | GCG | GCC | GCC | CTG | GCT | CCG | ACT | (C)AG | AC(C) | CGC | GCG | G | 3' |
|----|-----|-----|-----|-----|-----|-----|-----|-----|-----|-----|-----|-----|-----|-----|---|----|
|    | LEU | LEU | LEU | ALA | ALA | ALA | LEU | ALA | PRO | THR | GLN | THR | ARG | ALA | GLY |  |
|    | 8 | 9 | 10 | 11 | 12 | 13 | 14 | 15 | 16 | 17 | 18 | 19 | 20 | 21 | 22 |  |

Wild-type sequence

| GCC | GGC | TGG | CTC | CGA | CTC | AGA | CCC | GCG | CGG | GCC | CAC | ACT | CGC | TGA |
|-----|-----|-----|-----|-----|-----|-----|-----|-----|-----|-----|-----|-----|-----|-----|
| ALA | GLY | TRP | LEU | ARG | LEU | ARG | PRO | ALA | LEU | ALA | HIS | THR | ARG | STP |
| 13 | 14 | 15 | 16 | 17 | 18 | 19 | 20 | 21 | 22 | 23 | 24 | 25 | 26 | 27 |

Frameshift mutant sequence

0 258 899

Fig 2

NdeI      EcoRI    HindIII

0.52KB     RSV LTR inserted at NruI site

0.34KB    subcloned into M13mp9 for mutagenesis

NruI

HindII   NruI   SmaI   SmaI    BamHI     BamHI   HindII

1.0     2.0     3.0     4.0     5.0    KB

0 258 899

FIG. 3

Fig. 4

L-cell clones 293 transients
Mouse promoter ( wt ) ( RSV promoter )

322
HinfI  L          RSV
( frameshift )    wt    Amb    Amb+su⁺

← Input probe
   750nt

← 90nt protected

← 50nt protected

RSV CAP

EcoRI | Mouse CAP                    Mutations

BamHI                                Not I
5'                                              3'

────────────────────────────────── Input probe
                                    ( 750nt )
RSV CAP -
90 nt protected

Mouse CAP -
50 nt protected

Figure 5

Fig. 6

Figure 7

A: H-2K[b] positive L cells

B: H-2K[b] positive L cells diluted in a 10 fold excess of H-2K[b] negative L cells.

A: H-2K[b] positive L cells

B: H-2K[b] positive L cells diluted in a 100 fold excess of H-2K[b] negative L cells.

Fig. 8

L
LΔ2
LΔ5
LΔ6
LΔ10
Lam 4
Lam 11
Lam 13
Lam 14
Lam 15
Lam 19
Lam 21

10 copies
1 copy

+ 2.3 KB BamHI fragment containing RSV LTR sequences.

Fig. 9A

Fig. 9B

Fig. 10

**p291** is a 10.041 kbp plasmid which encodes the gene for EBNA-1 using the SV40 promoter, has OriP, and confers Hygromycin resistance.

SV40 early region 5171-346 HpaII to Hind III site ligated to the Cla I to Hind III of pBR322

EBV EBNA107930-110493 Hind III to Hind III (linkers were inserted into the Sau 3A site immediately before the EBNA start site) (Baer, et al., Nature 310:1984).

EBV ORI P 7338-9517 SphI to SstII sites blunt end ligated into BstE II site. (Sugden, et al., MCB 1985).

HSV TK regulatory region (McKnight, et.al., Cold Spring Harbor Symp. Quant. Biol. ,1977) PvuII fragment ligated into the poisonless pBR322 at Nae I sites. These sites lost in cloning.

HPH GENE(Gritz and Davies, Gene, 1983) BamHI fragment blunt-end ligated into SmaI to Bgl II sites in HSV TK sequences.

pBR322 poisonless vector (deletion of 1.1 kb in pBR322) confers ampicillin resistance. (Lusky & Botchan, Nature 293:1981.)

Fig. 11

0 258 899

Fig. 12

Fig. 13

0 258 899